(19) Europäisches Patentamt
European Patent Office
Office européen des brevets

(11) Veröffentlichungsnummer: **0 302 365**
**A2**

(12) # EUROPÄISCHE PATENTANMELDUNG

(21) Anmeldenummer: **88112044.8**

(22) Anmeldetag: **26.07.88**

(51) Int. Cl.⁴: **C07D 213/50 , C07D 213/30 , C07D 213/32 , C07D 405/04 , C07D 213/57 , C07D 405/12 , C07D 213/53 , C07D 213/55 , C07D 213/34 , A01N 43/40 , //C07C47/277**

(30) Priorität: **05.08.87 DE 3725968**

(43) Veröffentlichungstag der Anmeldung:
**08.02.89 Patentblatt 89/06**

(84) Benannte Vertragsstaaten:
**BE CH DE FR GB IT LI NL SE**

(71) Anmelder: **BAYER AG**
**Konzernverwaltung RP Patentabteilung**
**D-5090 Leverkusen 1 Bayerwerk(DE)**

(72) Erfinder: **Elbe, Hans-Ludwig, Dr.**
**Dasnöckel 49**
**D-5600 Wuppertal 11(DE)**
Erfinder: **Brandes, Wilhelm, Dr.**
**Eichendorffstrasse 3**
**D-5653 Leichlingen(DE)**
Erfinder: **Dutzmann, Stefan, Dr.**
**Leinenweberweg 33**
**D-4000 Düsseldorf 13(DE)**
Erfinder: **Hänssler, Gerd, Dr.**
**Am Arenzberg 58a**
**D-5090 Leverkusen 3(DE)**

(54) **Substituierte Pyridine.**

(57) Substituierte Pyridine der allgemeinen Formel (I),

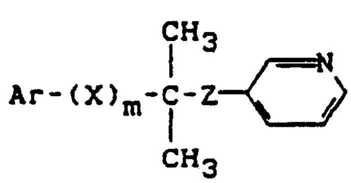

(I)

in welcher
die Substituenten die in der Beschreibung gegebene Bedeutung haben, und ihre Verwendung zur Bekämpfung von Schädlingen.

Die neuen substituierten Pyridine der allgemeinen Formel (I) können nach Analogieverfahren hergestellt werden, indem man z.B. geeignete Carbonsäureester mit Pyridyl-Grignard-Verbindungen umsetzt oder geeignete 3-substituierte Pyridine mit geeigneten Aldehyden umsetzt oder reduziert, alkyliert oder acyliert.

EP 0 302 365 A2

# Substituierte Pyridine

Die Erfindung betrifft neue substituierte Pyridine, mehrere Verfahren zu ihrer Herstellung sowie ihre Verwendung als Schädlingsbekämpfungsmittel.

Es ist bereits bekannt, daß bestimmte substituierte Pyridin-Derivate wie beispielsweise das 2-(4-Chlorphenoxy)-1-(2,4-dichlorphenyl)-1-(3-pyridyl)-ethanol oder das 1-(4-Chlor-2-methyl-phenoxy)-3,3-dimethyl-2-(3-pyridyl)-2-butanol oder das 3,3-Dimethyl-1-(2-methylphenoxy)-2-(3-pyridyl)-2-butanol oder das 1-(2,4-Dichlorphenoxy)-3,3-dimethyl-2-(3-pyridyl)-2-butanol oder deren pflanzenverträgliche Säureadditionssalze wie beispielsweise deren Naphthalin-1,5-disulfonate fungizide Eigenschaften besitzen (vgl. EP 1399).

Die Wirksamkeit dieser vorbekannten Verbindungen ist jedoch insbesondere bei niedrigen Aufwandmengen und Konzentrationen nicht in allen Anwendungsbereichen völlig zufriedenstellend.

Es wurden neue substituierte Pyridine der allgemeinen Formel (I),

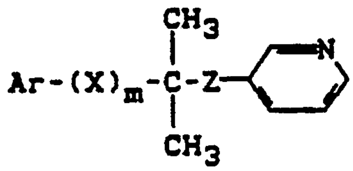

(I)

in welcher

Ar für gegebenenfalls substituiertes Aryl steht,

X für Sauerstoff, Schwefel, Sulfinyl, Sulfonyl oder für eine der Gruppen $-CH_2-$; $-O-CH_2-$; $-CH_2-O-$; $-O-CH_2-CH_2-$; $-S(O)_n-CH_2-$; $-CH_2-S(O)_n-$ oder $-S(O)_n-CH_2-CH_2-$ steht,

wobei

n jeweils für eine Zahl 0, 1 oder 2 steht,

Z für eine der Gruppen $-\underset{\underset{O}{\|}}{C}-$ oder $-\underset{\underset{OR}{|}}{CH}-$ steht,

wobei

R für Wasserstoff, Alkyl, Alkenyl, Alkinyl, für gegebenenfalls substiuiertes Aralkyl oder für Alkanoyl steht und

m für eine Zahl 0 oder 1 steht,

wobei jedoch für den Fall, daß X für Sauerstoff steht, Ar nicht gleichzeitig für einfach oder zweifach durch Chlor substituiertes Phenyl oder für einfach durch Difluormethoxy substituiertes Phenyl steht,

sowie deren pflanzenverträgliche Säureadditionssalze und Metallsalzkomplexe gefunden.

Die Verbindungen der Formel (I) können gegebenenfalls als geometrische und/oder optische Isomere oder Isomerengemische unterschiedlicher Zusammensetzung vorliegen. Sowohl die reinen Isomeren als auch die Isomerengemische werden erfindungsgemäß beansprucht.

Weiterhin wurde gefunden, daß man die neuen substituierten Pyridine der allgemeinen Formel (I),

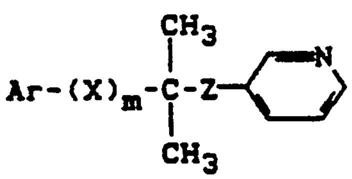

(I)

in welcher

Ar für gegebenenfalls substituiertes Aryl steht,

X für Sauerstoff, Schwefel, Sulfinyl, Sulfonyl oder für eine der Gruppen $-CH_2-$; $-O-CH_2-$; $-CH_2-O-$; $-O-CH_2-CH_2-$; $-S(O)_n-CH_2-$; $-CH_2-S(O)_n-$ oder $-S(O)_n-CH_2-CH_2-$ steht,

wobei

n jeweils für eine Zahl 0, 1 oder 2 steht,

Z für eine der Gruppen $-\underset{\underset{O}{\|}}{C}-$ oder $-\underset{\underset{OR}{|}}{CH}-$ steht,

wobei

R für Wasserstoff, Alkyl, Alkenyl, Alkinyl, für gegebenenfalls substituiertes Aralkyl oder für Alkanoyl steht

und
m für eine Zahl 0 oder 1 steht,
wobei jedoch für den Fall, daß X für Sauerstoff steht, Ar nicht gleichzeitig für einfach oder zweifach durch Chlor substituiertes Phenyl oder für einfach durch Difluormethoxy substituiertes Phenyl steht,
sowie deren pflanzenverträgliche Säureadditionssalze und Metallsalzkomplexe nach einem der im folgenden beschriebenen Verfahren erhält:

(a) Man erhält substituierte Pyridine der Formel (Ia),

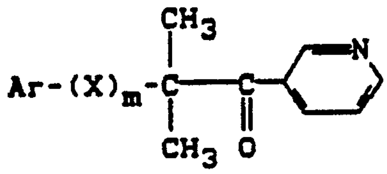

(Ia)

in welcher
Ar, X und m die oben angegebene Bedeutung haben,
wenn man Ester der Formel (II),

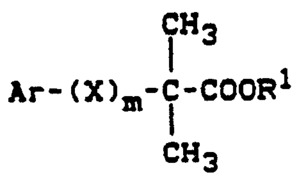

(II)

in welcher
$R^1$ für Alkyl steht und
Ar, X und m die oben angegebene Bedeutung haben,
mit Pyridyl-Grignard-Verbindungen der Formel (III),

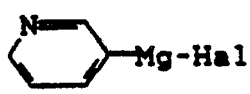

(III)

in welcher
Hal für Halogen steht,
gegebenenfalls in Gegenwart eines Verdünnungsmittels umsetzt;

(b) man erhält substituierte Pyridine der Formel (Ib),

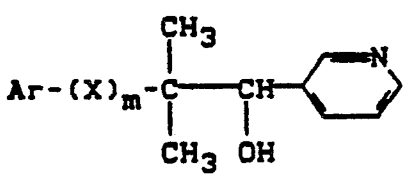

(Ib)

in welcher
Ar, X und m die oben angegebene Bedeutung haben,
wenn man Aldehyde der Formel (IV),

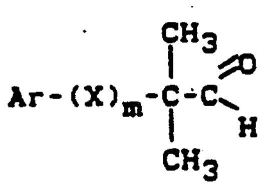

(IV)

in welcher
Ar, X und m die oben angegebene Bedeutung haben,
mit Pyridyl-Grignard-Verbindungen der Formel (III),

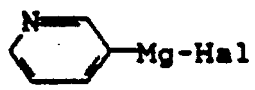

(III)

in welcher
Hal für Halogen steht,
gegebenenfalls in Gegenwart eines Verdünnungsmittels umsetzt;
(c) man erhält substituierte Pyridine der Formel (Ib),

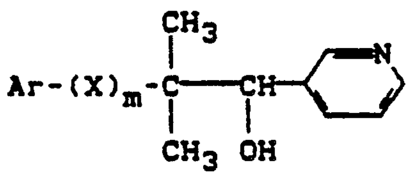

(Ib)

in welcher
Ar, X und m die oben angegebene Bedeutung haben,
alternativ auch, wenn man substituierte Pyridine der Formel (Ia),

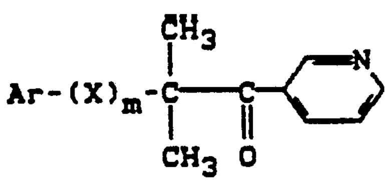

(Ia)

in welcher
Ar, X und m die oben angegebene Bedeutung haben,
mit einem Reduktionsmittel, gegebenenfalls in Gegenwart eines Verdünnungsmittels umsetzt;
(d) man erhält substituierte Pyridine der Formel (Ic),

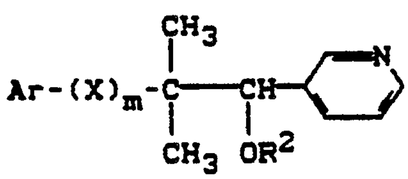

(Ic)

in welcher
$R^2$ für Alkyl, Alkenyl, Alkinyl, für gegebenenfalls substituiertes Aralkyl oder für Alkanoyl steht und
Ar, X und m die oben angegebene Bedeutung haben,
wenn man substituierte Pyridine der Formel (Ib),

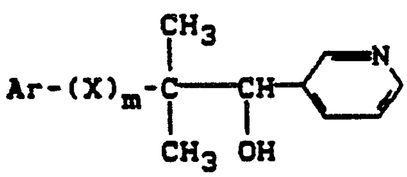

(Ib)

in welcher
Ar, X und m die oben angegebene Bedeutung haben,
mit Alkylierungs- oder Acylierungsmitteln der Formel (V),

$R^2$-A (V)

in welcher

A für eine elektronenanziehende Abgangsgruppe steht und

$R^2$ die oben angegebene Bedeutung hat,

gegebenenfalls in Gegenwart eines Verdünnungsmittels und gegebenenfalls in Gegenwart eines Reaktionshilfsmittels umsetzt

und gegebenenfalls anschließend eine Säure oder ein Metallsalz addiert.

Schließlich wurde gefunden, daß die neuen substituierten Pyridine der allgemeinen Formel (I) sowie deren Säureadditionssalze und Metallsalzkomplexe eine gute Wirksamkeit gegen Schädlinge insbesondere gegen pilzliche Schädlinge besitzen.

Überraschenderweise zeigen die erfindungsgemäßen substituierten Pyridin-Derivate der allgemeinen Formel (I) eine erheblich bessere fungizide Wirksamkeit als die aus dem Stand der Technik bekannten substituierten Pyridin-Derivate, wie beispielsweise das 2-(4-Chlorphenoxy)-1-(2,4-dichlorphenyl)-1-(3-pyridyl)-ethanol oder das 1-(4-Chlor-3-methyl-phenoxy)-3,3-dimethyl-2-(3-pyridyl)-2-butanol oder das 3,3-Dimethyl-1-(2-methylphenoxy)-2-(3-pyridyl)-2-butanol oder das 1-(2,4-Dichlorphenoxy)-3,3-dimethyl-2-(3-pyridyl)-2-butanol oder deren pflanzenverträgliche Säureadditionssalze wie beispielsweise deren Naphthalin-1,5-disulfonate, welches chemisch und wirkungsmäßig naheliegende Verbindungen sind.

Die erfindungsgemäßen substituierten Pyridine sind durch die Formel (I) allgemein definiert. Bevorzugt sind Verbindungen der Formel (I), bei welchen

Ar für gegebenenfalls einfach bis mehrfach, gleich oder verschieden substituiertes Phenyl steht, wobei als Substituenten infrage kommen: Halogen, Cyano, Nitro, jeweils geradkettiges oder verzweigtes Alkyl, Alkoxy, Alkylthio, Alkylsulfinyl oder Alkylsulfonyl mit jeweils 1 bis 4 Kohlenstoffatomen, geradkettiges oder verzweigtes, zweifach verknüpftes Alkandiyl mit 1 bis 10 Kohlenstoffatomen, Cycloalkyl mit 3 bis 7 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen, gegebenenfalls einfach bis mehrfach, gleich oder verschieden durch Halogen substituiertes, zweifach verknüpftes Dioxyalkylen mit 1 bis 6 Kohlenstoffatomen oder jeweils gegebenenfalls einfach bis mehrfach, gleich oder verschieden durch Halogen substituiertes Phenyl oder Phenoxy,

oder für gegebenenfalls einfach bis mehrfach, gleich oder verschieden substituiertes Naphthyl steht, wobei als Substituenten infrage kommen: Halogen, Cyano oder geradkettiges oder verzweigtes Alkyl mit 1 bis 4 Kohlenstoffatomen;

X für Sauerstoff, Schwefel, Sulfinyl, Sulfonyl oder für eine der Gruppen $-CH_2-$; $-O-CH_2-$; $-CH_2-O-$; $-O-CH_2-CH_2-$; $-S(O)_n-CH_2-$; $-CH_2-S(O)_n-$ oder $-S(O)_n-CH_2-CH_2-$ steht,

wobei

n jeweils für eine Zahl 0, 1 oder 2 steht,

Z für eine der Gruppen $-\underset{\underset{O}{\|}}{C}-$ oder $-\underset{\underset{OR}{|}}{CH}-$ steht,

wobei

R für Wasserstoff, für geradkettiges oder verzweigtes Alkyl mit 1 bis 6 Kohlenstoffatomen, für jeweils geradkettiges oder verzweigtes Alkenyl oder Alkinyl mit jeweils 3 bis 6 Kohlenstoffatomen oder für einfach bis mehrfach, gleich oder verschieden substituiertes Benzyl steht, wobei als Substituenten infrage kommen: Halogen, insbesondere Fluor, Chlor oder Brom, oder jeweils geradkettiges oder verzweigtes Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen oder für geradkettiges oder verzweigtes Alkanoyl mit 1 bis 7 Kohlenstoffatomen steht und

m für eine Zahl 0 oder 1 steht,

wobei jedoch für den Fall, daß X für Sauerstoff steht, Ar nicht gleichzeitig für einfach oder zweifach durch Chlor substituiertes Phenyl oder für einfach durch Difluormethoxy substituiertes Phenyl steht.

Besonders bevorzugt sind substituierte Pyridine der Formel (I), bei welchen

Ar für gegebenenfalls ein- bis dreifach, gleich oder verschieden substituiertes Phenyl steht, wobei als Substituenten infrage kommen: Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Ethylthio, Propan-1,3-diyl, Butan-1,4-diyl, Pentan-1,5-diyl, Dimethylpropan-1,3-diyl, Tetramethylpropan-1,3-diyl, Dimethylbutan-1,4-diyl, Tetramethylbutan-1,4-diyl, Cyclopropyl, Cyclobutyl, Cyclopentyl, Cylohexyl, Trifluormethyl, Dichlorfluormethyl, Difluorchlormethyl, Difluorbrommethyl, Trichlormethyl, Fluormethyl, Difluormethyl, Trifluorethyl, Tetrafluorethyl, Trifluorchlorethyl, Pentafluorethyl, Trifluormethoxy, Difluormethoxy, Fluormethoxy, Dichlorfluormethoxy, Difluorchlormethoxy, Difluorbrommethoxy, Trichlormethoxy, Trifluorethoxy, Tetrafluorethoxy, Pentafluorethoxy, Trifluorchlorethoxy, Trifluordichlorethoxy, Difluortrichlorethoxy, Pentachlorethoxy, Trifluormethylthio, Difluormethylthio, Fluormethylthio, Difluorchlormethylthio, Dichlorfluormethylthio, Difluorethylthio, Difluorbrommethylthio, Trichlormethylthio, Trifluorethylthio, Tetrafluorethylthio, Pentafluorethylthio, Trifluorchlorethylthio, Trifluordichlorethylthio, Pentachlorethylthio, Methylsulfinyl, Trifluormethylsulfinyl, Dichlorfluormethylsulfinyl, Difluorchlormethylsulfinyl, Fluormethylsulfinyl, Difluormethylsulfinyl, Methylsulfonyl, Trifluormethylsulfonyl, Dichlorfluormethylsulfonyl, Di fluorchlormethylsulfonyl, Fluormethylsulfonyl, Difluormethylsulfonyl, Methoxycarbonyl, Ethoxycarbonyl, Methoximinomethyl, Ethoximinomethyl, Methoximinoethyl, Ethoximinoethyl, Difluordioxymethylen, Tetrafluordioxyethylen, Trifluordioxyethylen, Difluordioxyethylen, Dioxymethylen, Dioxyethylen, jeweils gegebenenfalls ein- bis dreifach durch Fluor oder Chlor substituiertes Phenyl oder Phenoxy;

oder für gegebenenfalls ein- bis dreifach, gleich oder verschieden substituiertes α-Naphthyl oder β-Naphthyl steht, wobei als Substituenten jeweils infrage kommen: Fluor, Chlor, Brom, Cyano, Methyl, Ethyl, n- oder i-Propyl;

X für Sauerstoff, Schwefel, Sulfinyl, Sulfonyl oder für eine der Gruppen $-CH_2-$; $-O-CH_2-$; $-CH_2-O-$; $-O-CH_2-CH_2-$; $-S(O)_n-CH_2-$; $-CH_2-S(O)_n-$ oder $-S(O)_n-CH_2-CH_2-$ steht,

wobei

n jeweils für eine Zahl 0, 1 oder 2 steht,

Z für eine der Gruppen $-\underset{\underset{O}{\|}}{C}-$ oder $-\underset{\underset{OR}{|}}{C}H-$ steht,

wobei

R für Wasserstoff, Methyl, Ethyl, n- oder i-Propyl, n-, i-, s- oder t-Butyl, Allyl, n-oder i-Butenyl, Propargyl, n- oder i-Butinyl, für gegebenenfalls ein- bis dreifach, gleich oder verschieden durch Fluor, Chlor, Brom, Methyl, Ethyl, n- oder i-Propyl, n-, i-, s-oder t-Butyl, Methoxy, Ethoxy, n- oder i-Propoxy, Methylthio, Ethylthio, Trifluormethyl, Trifluormethoxy oder Trifluormethylthio substituiertes Benzyl oder für Acetyl oder Propionyl steht und

m für eine Zahl 0 oder 1 steht,

wobei jedoch für den Fall, daß X für Sauerstoff steht, Ar nicht gleichzeitig für einfach oder zweifach durch Chlor substituiertes Phenyl oder für einfach durch Difluormethoxy substituiertes Phenyl steht.

Ganz besonders bevorzugt sind substituierte Pyridine der Formel (I), bei welchen

Ar für gegebenenfalls ein- bis dreifach, gleich oder verschieden substituiertes Phenyl steht, wobei als Substituenten infrage kommen: Fluor, Chlor, Brom, Cyano, Nitro, Methyl, Ethyl, t-Butyl, Methoxy, Ethoxy, n- oder i-Propoxy, Methylthio, Cyclopropyl, Cyclopentyl, Cyclohexyl, Propan-1,3-diyl, Butan-1,4-diyl, Pentan-1,5-diyl, 1,1,3,3-Tetramethylpropan-1,3-diyl, Trifluormethyl, Trifluormethoxy, Trifluormethylthio, Difluormethyl, Difluormethoxy, Difluormethylthio, Difluorchlormethyl, Difluorchlormethoxy, Difluorchlormethylthio, Dichlorfluormethyl, Dichlorfluormethoxy, Dichlorfluormethylthio, Methoximinomethyl, Ethoximinomethyl, Methoximinoethyl, Ethoximinoethyl, Difluordioxymethylen, Trifluordioxyethylen, Tetrafluordioxyethylen, Phenoxy oder Phenyl außerdem für α-Naphthyl oder β-Naphthyl steht,

X für Schwefel, für eine $-CH_2$-Gruppe, eine $-O-CH_2$-Gruppe, eine $-S-CH_2$-Gruppe, eine $-O-CH_2-CH_2$-Gruppe oder eine $-S-CH_2-CH_2$-Gruppe steht,

Z für eine $-\underset{\underset{O}{\|}}{C}-$ Gruppe oder eine $-\underset{\underset{OH}{|}}{C}H-$ Gruppe steht

und

m für eine Zahl 0 oder 1 steht.

Ganz besonders bevorzugt sind daneben auch substituierte Pyridine der Formel (I), bei welchen

Ar für ein- bis dreifach, gleich oder verschieden substituiertes Phenyl steht, wobei mindestens ein Substituent für einen der folgenden Reste steht: Methylthio, Difluormethylthio, Trifluormethylthio, Difluorchlormethylthio, Dichlorfluormethylthio, Propan-1,3-diyl, Butan-1,4-diyl, Pentan-1,5-diyl, 1,1,3,3-Tetramethylpropan-1,3-diyl, Cyclopropyl, Cyclopentyl, Cyclohexyl, Dioxymethylen, Dioxyethylen, Difluordioxymethylen, Trifluordioxyethylen, Tetrafluordioxyethylen, Methoximinomethyl, Methoximinoethyl, Ethoximinomethyl, Ethoximinoethyl, gegebenenfalls ein- bis dreifach durch Fluor und/oder Chlor substituiertes Phenoxy oder ein- bis dreifach durch Fluor und/oder Chlor substituiertes Phenyl; und wobei als weitere Substituenten infrage kommen: Fluor, Chlor, Brom, Cyano, Nitro, Methyl, Ethyl, t-Butyl, Methoxy, Ethoxy, n- oder i-Propoxy, Trifluormethyl, Trifluormethoxy, Difluormethyl, Difluormethoxy, Difluorchlormethyl, Difluorchlormethoxy, Dichlorfluormethyl, Dichlorfluormethoxy oder Phenyl,

außerdem für α-Naphthyl oder β-Naphthyl steht,

X für Sauerstoff steht,

Z für eine $-\underset{\underset{O}{\|}}{C}-$-Gruppe oder eine $-\underset{\underset{OH}{|}}{C}H-$-Gruppe steht

und
m für eine Zahl 0 oder 1 steht.

Bevorzugte erfindungsgemäße Verbindungen sind auch Additionsprodukte aus Säuren und denjenigen substituierten Pyridinen der Formel (I), in denen die Substituenten Ar, X, m und Z die Bedeutung haben, die bereits vorzugsweise für diese Substituenten genannt wurden.

Zu den Säuren, die addiert werden können, gehören vorzugsweise Halogenwasserstoffsäuren, wie z.B. Chlorwasserstoffsäure und Bromwasserstoffsäure, insbesondere Chlorwasserstoffsäure, ferner Phosphorsäure, Salpetersäure und Schwefelsäure, mono-, bi- und trifunktionelle Carbonsäuren und Hydroxycarbonsäuren, wie z.B. Essigsäure, Maleinsäure, Bernsteinsäure, Fumarsäure, Weinsäure, Zitronensäure, Salicylsäure, Sorbinsäure und Milchsäure, Sulfonsäuren, wie p-Toluolsulfonsäure, 1,5-Naphthalindisulfonsäure, sowie Saccharin oder Thiosaccharin.

Außerdem bevorzugte erfindungsgemäße Verbindungen sind Additionsprodukte aus Salzen von Metallen der II. bis IV. Haupt- und der I. und II. sowie IV. bis VIII. Nebengruppe und denjenigen substituierten Pyridinen der Formel (I), in denen die Substituenten Ar, X, m und Z die Bedeutungen haben, die bereits vorzugsweise für diese Substituenten genannt wurden.

Hierbei sind Salze des Kupfers, Zinks, Mangans, Magnesiums, Zinns, Eisens und des Nickels besonders bevorzugt. Als Anionen dieser Salze kommen solche in Betracht, die sich von solchen Säuren ableiten, die zu pflanzenverträglichen Additionsprodukten führen. Besonders bevorzugte derartige Säuren sind in diesem Zusammenhang die Halogenwasserstoffsäuren, wie z.B. die Chlorwasserstoffsäure und die Bromwasserstoffsäure, ferner Phosphorsäure, Salpetersäure und Schwefelsäure.

Im einzelnen seien außer den bei den Herstellungsbeispielen genannten Verbindungen die folgenden substituierten Pyridine der allgemeinen Formel (I) genannt:

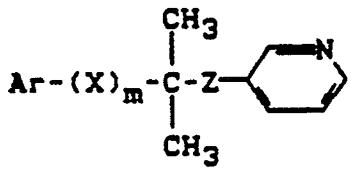

(I)

| Ar | $-(X)_m-$ | Z |
| --- | --- | --- |
| 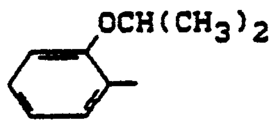 | $-O-CH_2-$ | 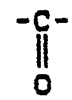 |
| 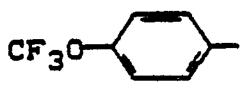 | $-O-CH_2-$ | 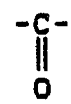 |
| 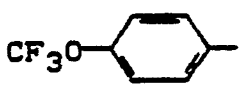 | $-O-CH_2-$ | 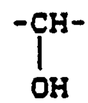 |
| 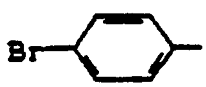 | $-O-CH_2-$ | 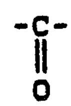 |

| Ar | $-(X)_m-$ | Z |
|---|---|---|
| 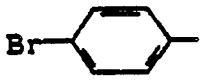 | $-O-CH_2-$ | 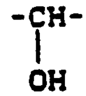 |
| 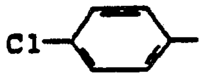 | $-O-CH_2-$ | 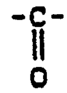 |
| 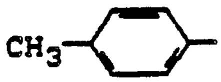 | $-O-CH_2-$ | 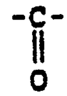 |
| 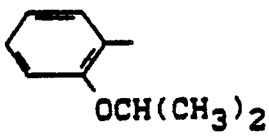 | $-O-CH_2-$ | 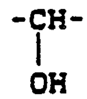 |
| 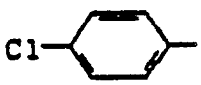 | $-O-CH_2-$ | 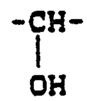 |
| 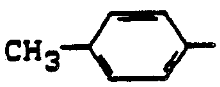 | $-O-CH_2-$ | 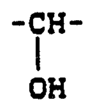 |
| 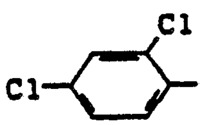 | $-O-CH_2-$ | 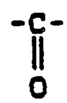 |

| Ar | $-(X)_m-$ | Z |
|---|---|---|
| 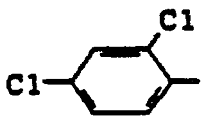 | $-O-CH_2-$ | 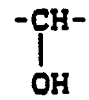 |
| 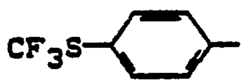 | $-O-CH_2-$ | 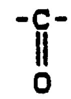 |
| 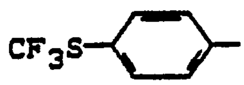 | $-O-CH_2-$ | 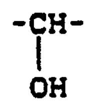 |
| 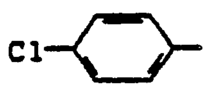 | - | 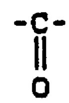 |
| 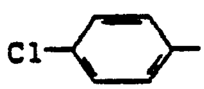 | - | 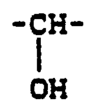 |
| 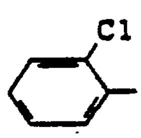 | - | 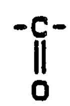 |
| 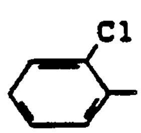 | - | 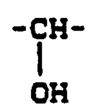 |

| Ar | $-(X)_m-$ | Z |
|---|---|---|
| 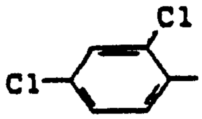 | - | 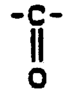 |
| 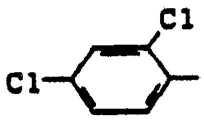 | - | 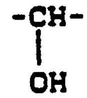 |
| 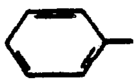 | - | 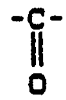 |
| 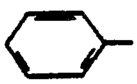 | - | 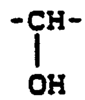 |
| 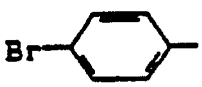 | - | 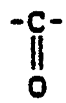 |
| 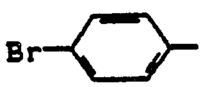 | - | 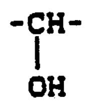 |
| 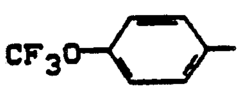 | - | 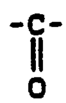 |

| Ar | $-(X)_m-$ | Z |
|---|---|---|
| 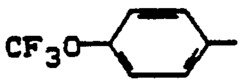 | - | 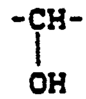 |
| 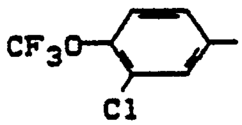 | - | 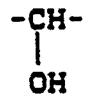 |
| 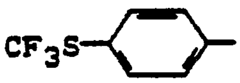 | - | 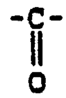 |
| 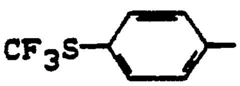 | - | 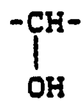 |
| 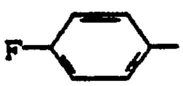 | - | 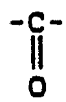 |
| 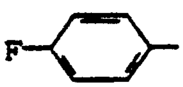 | - | 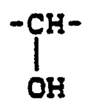 |
| 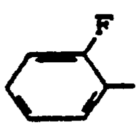 | - | 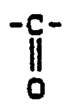 |

| Ar | $-(X)_m-$ | Z |
|---|---|---|
| 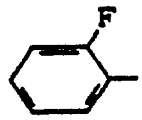 | - | 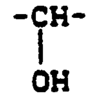 |
| 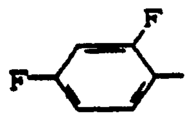 | - | 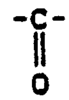 |
| 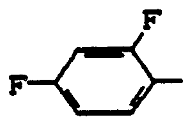 | - | 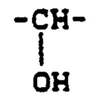 |
| 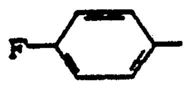 | $-O-CH_2-$ | 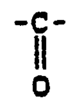 |
| 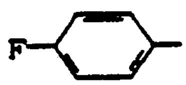 | $-O-CH_2-$ | 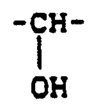 |
| 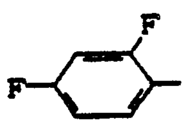 | $-O-CH_2-$ | 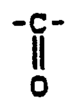 |
| 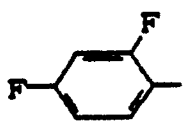 | $-O-CH_2-$ | 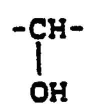 |

| Ar | $-(X)_m-$ | Z |
|---|---|---|
| 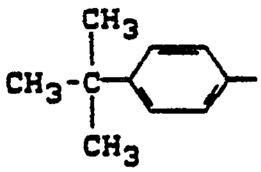 | $-O-CH_2-$ | 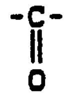 |
| 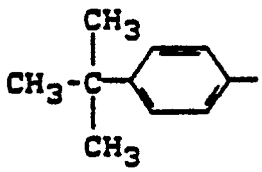 | $-O-CH_2-$ | 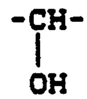 |
| 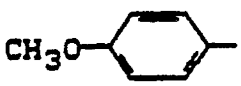 | $-O-CH_2-$ | 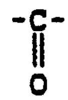 |
| 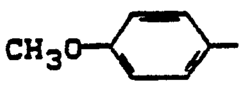 | $-O-CH_2-$ | 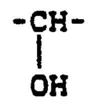 |
| 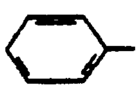 | $-O-CH_2-$ | 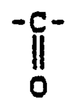 |
| 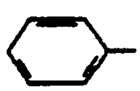 | $-O-CH_2-$ | 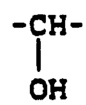 |
| 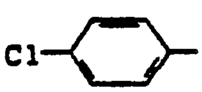 | $-S-CH_2-$ | 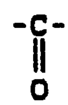 |

| Ar | $-(X)_m-$ | Z |
|---|---|---|
| 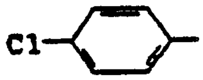 | $-S-CH_2-$ | 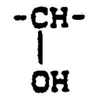 |
| 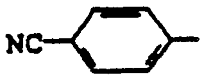 | $-S-CH_2-$ | 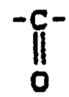 |
| 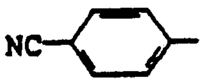 | $-S-CH_2-$ | 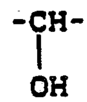 |
| 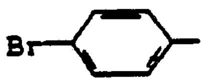 | $-S-CH_2-$ | 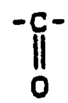 |
| 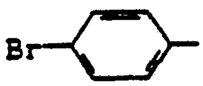 | $-S-CH_2-$ | 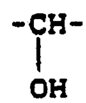 |
| 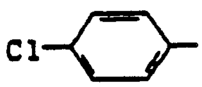 | $-O-CH_2-CH_2-$ | 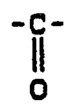 |
| 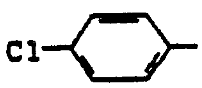 | $-O-CH_2-CH_2-$ | 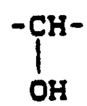 |

| Ar | $-(X)_m-$ | Z |
|---|---|---|
| 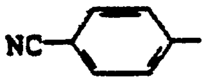 | S | 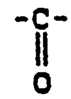 |
| 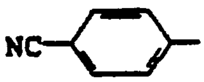 | S | 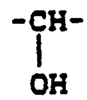 |
| 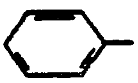 | S | 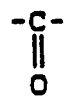 |
| 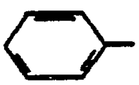 | S | 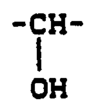 |
| 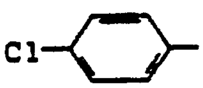 | S | 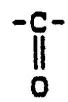 |
| 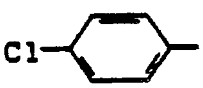 | S | 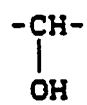 |
| 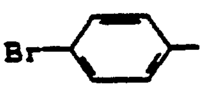 | S | 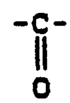 |

| Ar | $-(X)_m-$ | Z |
|---|---|---|
| 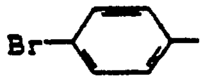 | S | 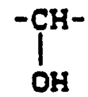 |
| 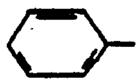 | $-S-CH_2-$ | 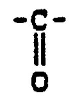 |
| 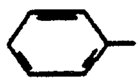 | $-S-CH_2-$ | 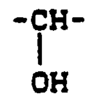 |
| 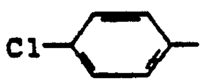 | $-S-CH_2-CH_2-$ | 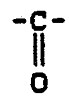 |
| 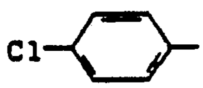 | $-S-CH_2-CH_2-$ | 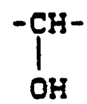 |
| 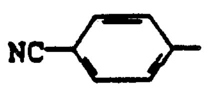 | $-S-CH_2-CH_2-$ | 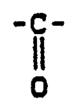 |
| 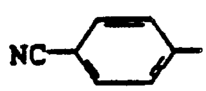 | $-S-CH_2-CH_2-$ | 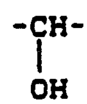 |

| Ar | $-(X)_m-$ | Z |
|---|---|---|
| 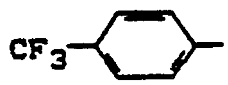 | S | CO |
| 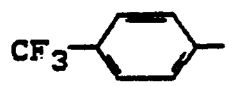 | S | 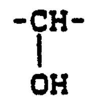 |
| 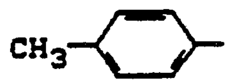 | $-O-CH_2-$ | 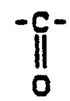 |
| 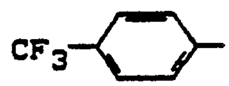 | $-O-CH_2$ | 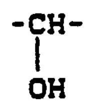 |
| 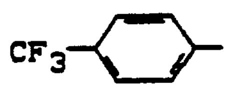 | $-S-CH_2-$ | 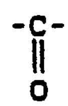 |
| 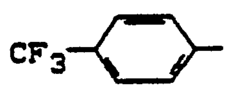 | $-S-CH_2-$ | 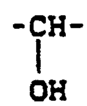 |
| 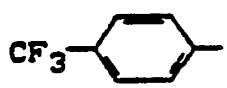 | $-CH_2-$ | 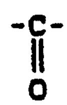 |
| 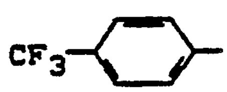 | $-CH_2-$ | 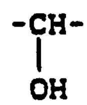 |

| Ar | $-(X)_m-$ | Z |
|---|---|---|
| 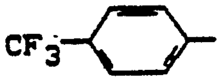 | - | 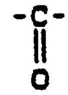 |
| 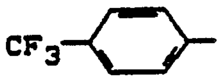 | - | 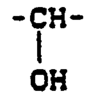 |
| 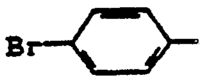 | $-CH_2-$ | 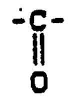 |
| 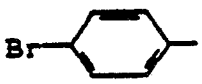 | $-CH_2-$ | 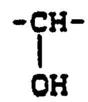 |
| 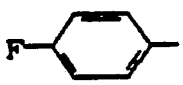 | $-CH_2-$ | 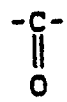 |
| 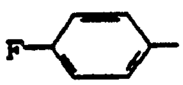 | $-CH_2-$ | 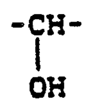 |
| 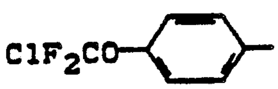 | $-CH_2-$ | 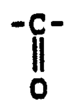 |

| Ar | $-(X)_m-$ | Z |
|---|---|---|
| 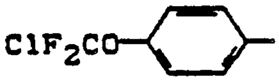 | $-CH_2-$ | 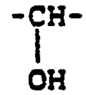 |
| 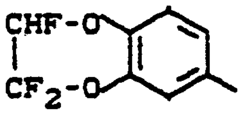 | $-CH_2-$ | 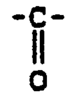 |
| 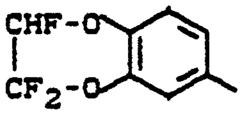 | $-CH_2-$ | 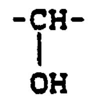 |
| 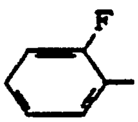 | $-CH_2-$ | 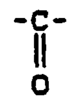 |
| 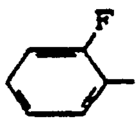 | $-CH_2-$ | 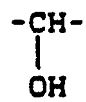 |
| 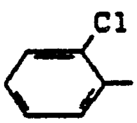 | $-CH_2-$ | 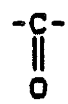 |
| 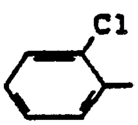 | $-CH_2-$ | 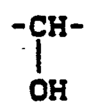 |

| Ar | $-(X)_m-$ | Z |
|---|---|---|
| 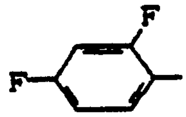 | $-CH_2-$ | 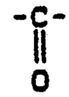 |
| 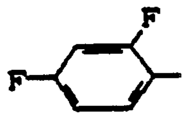 | $-CH_2-$ | 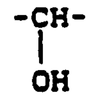 |
| 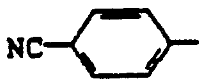 | $-CH_2-$ | 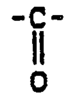 |
| 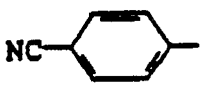 | $-CH_2-$ | 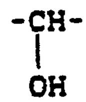 |
| 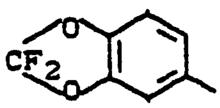 | $-CH_2-$ | 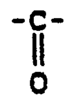 |
| 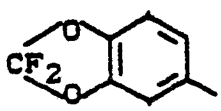 | $-CH_2-$ | 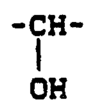 |
| 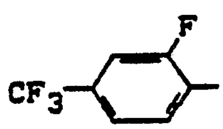 | $-CH_2-$ | 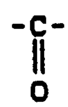 |

| Ar | $-(X)_m-$ | Z |
|---|---|---|
| 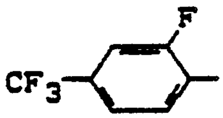 | $-CH_2-$ | 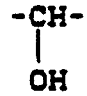 |
| 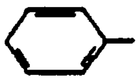 | $-CH_2-$ | 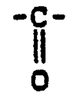 |
| 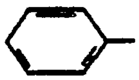 | $-CH_2-$ | 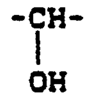 |
| 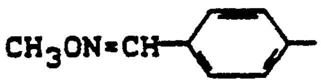 | -O- | 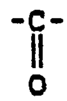 |
| 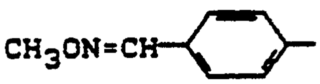 | -O- | 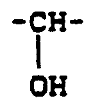 |
| 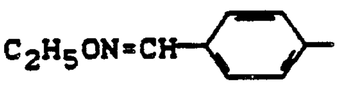 | -O- | 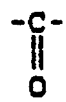 |
| 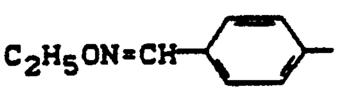 | -O- | 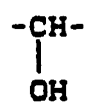 |

| Ar | -(X)$_m$- | Z |
|---|---|---|
| 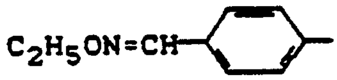 | -O- | 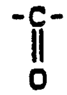 |
| 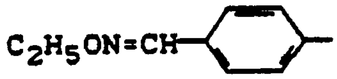 | -O- | 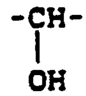 |
| 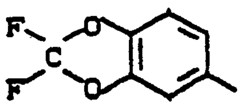 | -O- | 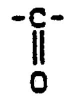 |
| 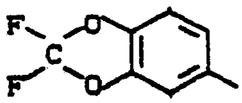 | -O- | 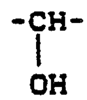 |
| 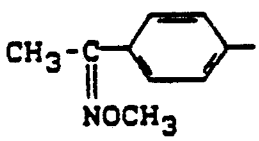 | -O- | 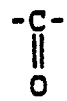 |
| 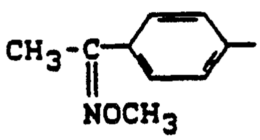 | -O- | 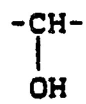 |
| 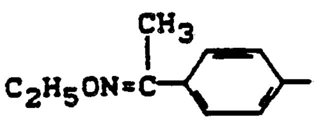 | -O- | 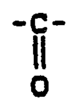 |

| Ar | $-(X)_m-$ | Z |
|---|---|---|
| 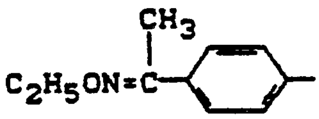 | -O- | 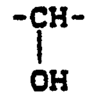 |
| 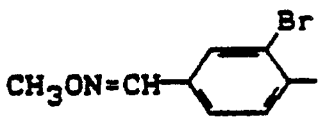 | -O- | 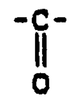 |
| 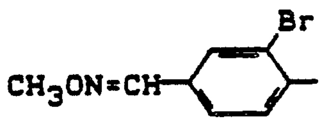 | -O- | 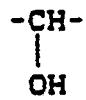 |
| 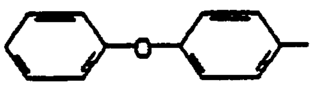 | -O- | 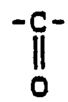 |
| 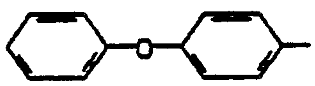 | -O- | 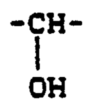 |
| 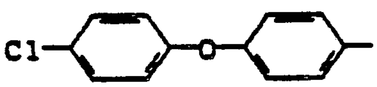 | -O- | 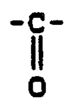 |
| 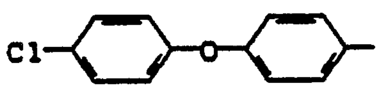 | -O- | 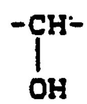 |

| Ar | -(X)$_m$- | Z |
|---|---|---|
| 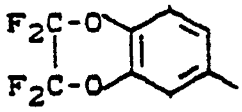 | -O- | 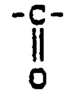 |
| 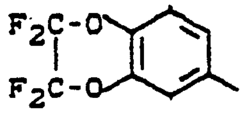 | -O- | 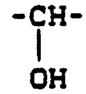 |
| 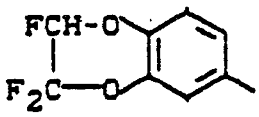 | -O- | 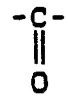 |
| 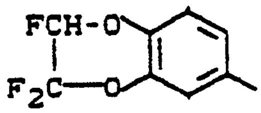 | -O- | 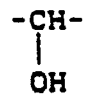 |
| 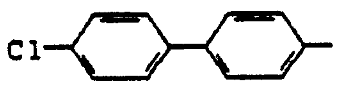 | -O- | 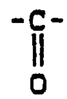 |
| 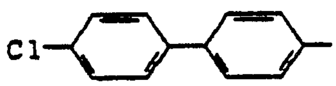 | -O- | 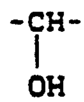 |
| 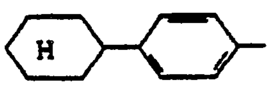 | -O- | 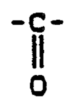 |

| Ar | $-(X)_m-$ | Z |
|---|---|---|
| 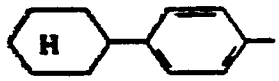 | -O- | 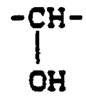 |
| 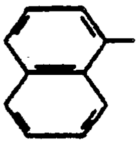 | -O- | 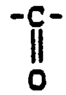 |
| 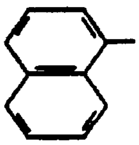 | -O- | 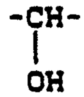 |
| 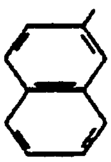 | -O- | 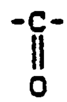 |
| 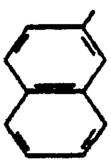 | -O- | 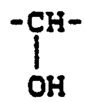 |
| 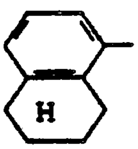 | -O- | 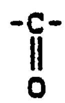 |
| 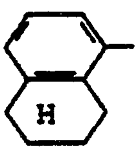 | -O- | 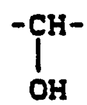 |

| Ar | $-(X)_m-$ | Z |
|---|---|---|
| 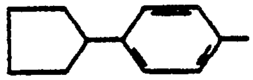 | -O- | 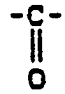 |
| 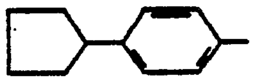 | -O- | 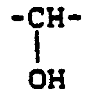 |
| 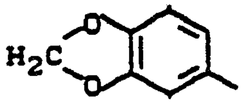 | -O- | 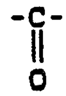 |
| 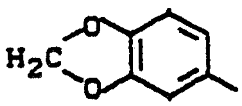 | -O- | 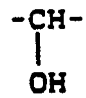 |
| 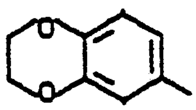 | -O- | 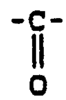 |
| 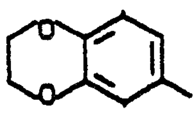 | -O- | 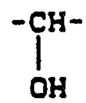 |
| 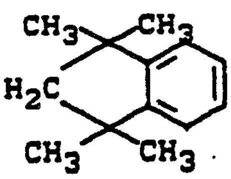 | -O- | 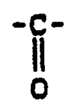 |

| Ar | $-(X)_m-$ | Z |
|---|---|---|
| 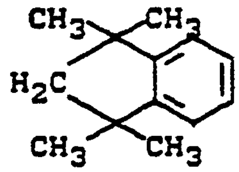 | -O- | 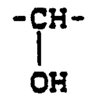 |
| 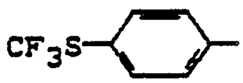 | -O- | 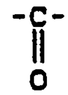 |
| 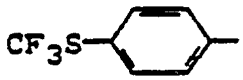 | -O- | 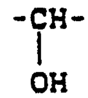 |
| 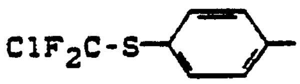 | -O- | 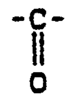 |
| 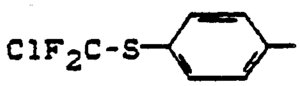 | -O- | 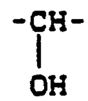 |
| 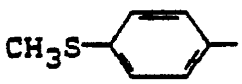 | -O- | 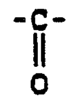 |
| 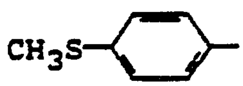 | -O- | 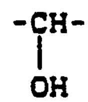 |

Verwendet man beispielsweise 2,2-Dimethyl-3-(4-chlorphenyl)-propionsäuremethylester und 3-Pyridylmagnesiumbromid als Ausgangsstoffe, so läßt sich der Reaktionsablauf des erfindungsgemäßen Verfahrens (a) durch folgendes Formelschema darstellen:

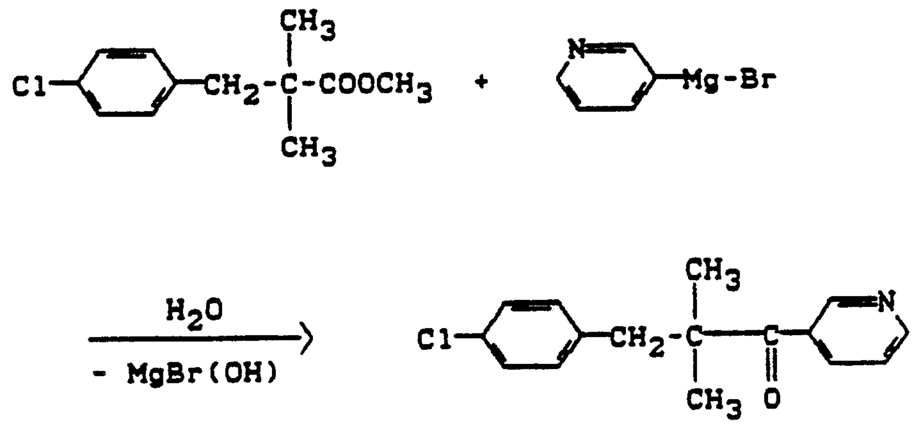


Verwendet man beispielsweise 2-(2,4-Dichlorphenylthio)-isobutyraldehyd und 3-Pyridylmagnesiumbromid als Ausgangsstoffe, so läßt sich der Reaktionsablauf des erfindungsgemäßen Verfahrens (b) durch das folgende Formelschema darstellen:

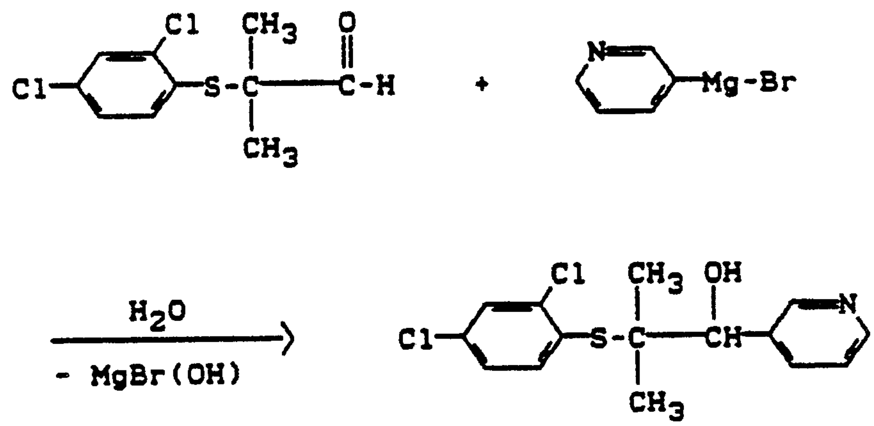


Verwendet man beispielsweise 3-(2,4-Dichlorphenoxy)-2,2-dimethyl-1-(3-pyridyl)-propan-1-on und Natriumborhydrid als Ausgangsstoffe, so läßt sich der Reaktionsablauf des erfindungsgemäßen Verfahrens (c) durch das folgende Formelschema darstellen:

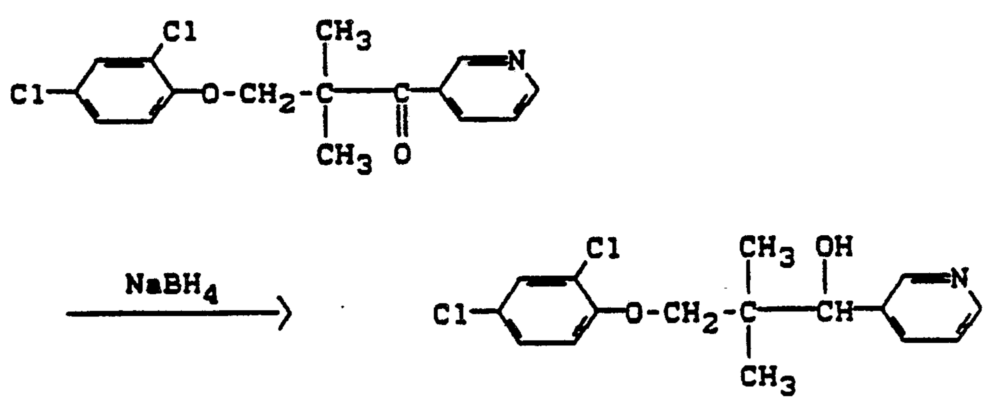


Verwendet man beispielsweise 4-(4-t-Butylphenoxy)-2,2-dimethyl-1-(3-pyridyl)-butan-1-ol und Dimethylsulfat als Ausgangsstoffe, so läßt sich der Reaktionsablauf des erfindungsgemäßen Verfahrens (d) durch das folgende Formelschema darstellen:

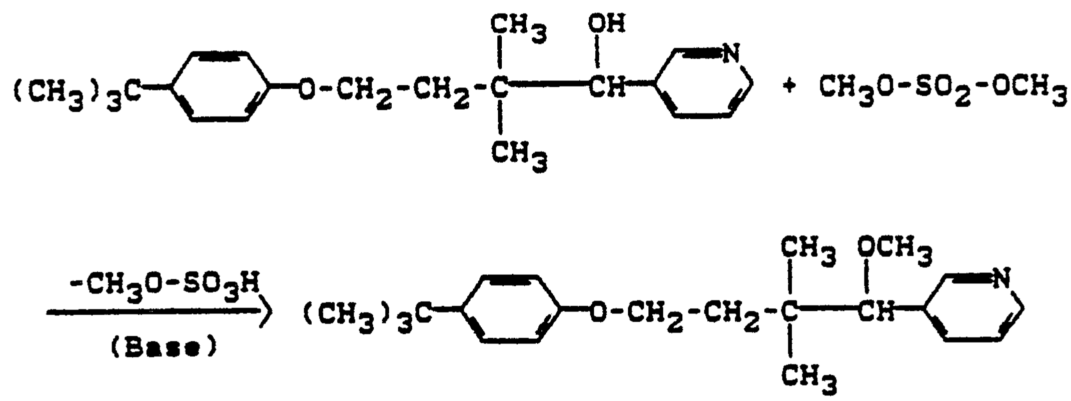


Die zur Durchführung des erfindungsgemäßen Verfahrens (a) als Ausgangsstoffe benötigten Ester sind durch die Formel (II) allgemein definiert. In dieser Formel (II) stehen Ar, X und m vorzugsweise für diejenigen Reste, die bereits im Zusammenhang mit der Beschreibung der erfindungsgemäßen Stoffe der Formel (I) als bevorzugt für diese Substituenten genannt wurden.

$R^1$ steht vorzugsweise für geradkettiges oder verzweigtes Alkyl mit 1 bis 4 Kohlenstoffatomen, insbesondere für Methyl oder Ethyl.

Die Ester der Formel (II) sind bekannt oder erhältlich in Analogie zu bekannten Verfahren (vgl. z.B. EP 221 844 oder Przem. Chem. 59, 495-498 [1980] bzw. CA 94: 102 975e sowie die Herstellungsbeispiele).

Die zur Durchführung des erfindungsgemäßen Verfahrens (b) als Ausgangsstoffe benötigten Aldehyde sind durch die Formel (IV) allgemein definiert. In dieser Formel (IV) stehen Ar, X und m vorzugsweise für diejenigen Reste, die bereits im Zusammenhang mit der Beschreibung der erfindungsgemäßen Stoffe der Formel (I) als bevorzugt für diese Substituenten genannt wurden.

Die Aldehyde der Formel (IV) sind ebenfalls bekannt oder erhältlich in Analogie zu bekannten Verfahren (vgl. z.B. J. Org. Chem. 40, 2282 [1975]; J. Org. Chem. 40, 3079 [1975]; Arm. Khim. Zh. 25, 861 [1972]; Brit. GB 1 140 014; Liebigs. Anm. Chem. 1979, 1585; DE 3 531 585; DE 2 056 589).

Die zur Durchführung der erfindungsgemäßen Verfahren (a) und (b) als Ausgangsstoffe benötigten Pyridyl-Grignard-Verbindungen sind durch die Formel (III) allgemein definiert. In dieser Formel (III) steht Hal vorzugsweise für Chlor, Brom oder Iod, insbesondere für Brom.

Die Pyridyl-Grignard-Verbindungen der Formel (III) sind ebenfalls bekannt (vgl. z.B. EP 221 844 oder Angew. Chem.; Int. Ed. Engl. 8, 279 [1969]).

Die zur Durchführung des erfindungsgemäßen Verfahrens (c) als Ausgangsstoffe benötigten substituierten Pyridine sind durch die Formel (Ia) allgemein definiert. In dieser Formel (Ia) stehen Ar, X und m vorzugsweise für diejenigen Reste, die bereits im Zusammenhang mit der Beschreibung der erfindungsgemäßen Stoffe der Formel (I) als bevorzugt für diese Substituenten genannt wurden.

Die substituierten Pyridin-Derivate der Formel (Ia) sind erfindungsgemäße Verbindungen und erhältlich mit Hilfe des erfindungsgemäßen Verfahrens (a).

Die zur Durchführung des erfindungsgemäßen Verfahrens (d) als Ausgangsstoffe benötigten substituierten Pyridine sind durch die Formel (Ib) allgemein definiert. In dieser Formel (Ib) stehen Ar, X und m vorzugsweise für diejenigen Reste, die bereits im Zu sammenhang mit der Beschreibung der erfindungsgemäßen Stoffe der Formel (I) als bevorzugt für diese Substituenten genannt wurden.

Die substituierten Pyridine der Formel (Ib) sind erfindungsgemäße Verbindungen und erhältlich mit Hilfe der erfindungsgemäßen Verfahren (b) und (c).

Die zur Durchführung des erfindungsgemäßen Verfahrens (d) weiterhin als Ausgangsstoffe benötigten Alkylierungs- oder Acylierungsmittel sind durch die Formel (V) allgemein definiert. In dieser Formel (V) steht $R^2$ vorzugsweise für geradkettiges oder verzweigtes Alkyl mit 1 bis 6 Kohlenstoffatomen, für jeweils geradkettiges oder verzweigtes Alkenyl oder Alkinyl mit jeweils 3 bis 6 Kohlenstoffatomen, für einfach bis mehrfach gleich oder verschieden substituiertes Benzyl, wobei als Substituenten infrage kommen: Halogen, insbesondere Fluor, Chlor oder Brom, oder jeweils geradkettiges oder verzweigtes Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen oder für geradkettiges oder verzweigtes Alkanoyl mit 1 bis 7 Kohlenstoffatomen. $R^2$ steht insbesondere für Methyl, Ethyl, n- oder i-Propyl, n-, i-, s- oder t-Butyl, Allyl, n- oder i-Butenyl, Propargyl, n- oder i-Butinyl, für gegebenenfalls ein- bis dreifach, gleich oder verschieden durch Fluor, Chlor, Brom, Methyl, Ethyl, n- oder i-Propyl, n-, i-, s- oder t-Butyl, Methoxy, Ethoxy, n- oder i-Propoxy, Methylthio, Ethylthio, Trifluormethyl, Trifluormethoxy oder Trifluormethylthio

substituiertes Benzyl oder für Acetyl oder Propionyl.

A steht vorzugsweise für Halogen, insbesondere für Chlor, Brom oder Iod oder im Fall der Alkylierungsmittel auch für gegebenenfalls substituiertes Alkoxysulfonyloxy oder Arylsulfonyloxy, wie beispielsweise Methoxysulfonyloxy oder p-Toluolsulfonyloxy oder im Fall der Acylierungsmittel auch für einen Rest

$$R^2-\underset{\underset{O}{\|}}{C}-O-,$$

wobei $R^2$ die oben angegebene Bedeutung hat.

Die Alkylierungsmittel oder Acylierungsmittel der Formel (V) sind allgemein bekannte Verbindungen der organischen Chemie.

Als Verdünnungsmittel zur Durchführung der erfindungsgemäßen Verfahren (a) und (b) kommen inerte organische Lösungsmittel infrage. Hierzu gehören insbesondere aliphatische, alicyclische oder aromatische Kohlenwasserstoffe, wie beispielsweise Benzin, Benzol, Toluol, Xylol, Petrolether, Hexan oder Cyclohexan, Ether, wie Diethylether, Diisopropylether, Dioxan, Tetrahydrofuran oder Ethylenglykoldimethyl- oder -diethylether, oder Amide, wie Hexamethylphosphorsäuretriamid.

Die Reaktionstemperaturen können bei der Durchführung der erfindungsgemäßen Verfahren (a) und (b) in einem größeren Bereich variiert werden. Im allgemeinen arbeitet man bei Temperaturen zwischen - 50 °C und + 150 °C, vorzugsweise bei Temperaturen zwischen - 20 °C und + 120 °C.

Zur Durchführung des erfindungsgemäßen Verfahrens (a) setzt man pro Mol an Keton der Formel (II) im allgemeinen 1.0 bis 3.0 Mol, vorzugsweise 1.0 bis 1.5 Mol an Pyridyl-Grignard-Verbindung der Formel (III) ein.

Zur Durchführung des erfindungsgemäßen Verfahrens (b) setzt man pro Mol an Aldehyd der Formel (IV) im allgemeinen 1.0 bis 3.0 Mol, vorzugsweise 1.0 bis 1.5 Mol an Pyridyl-Grignard-Verbindung der Formel (III) ein. Die Reaktionsdurchführung erfolgt sowohl bei Verfahren (a) als auch bei Verfahren (b) nach allgemein üblichen Methoden.
Dabei kann die Umsetzung erforderlichenfalls in Gegenwart eines geeigneten Inertgases, wie beispielsweise Stickstoff oder Helium durchgeführt werden.
Es ist auch möglich, die als Reaktionspartner eingesetzte Pyridyl-Grignard-Verbindung der Formel (III) aus geeigneten Ausgangsverbindungen, wie beispielsweise 3-Brompyridin und Isopropylmagnesiumbromid, in einer vorgelagerten Reaktion direkt im Reaktionsgefäß herzustellen und ohne Isolierung im Eintopfverfahren mit den Ketonen der Formel (II) bzw. mit den Aldehyden der Formel (IV) weiter umzusetzen.
Die Aufarbeitung und Isolierung der Reaktionsprodukte erfolgt sowohl bei Verfahren (a) als auch nach Verfahren (b) nach üblichen Methoden (vgl. Herstellungsbeispiele).

Als Reduktionsmittel zur Durchführung des erfindungsgemäßen Verfahrens (c) kommen alle üblicherweise für derartige Carbonylgruppenreduktionen verwendbaren Reduktionsmittel infrage. Vorzugsweise verwendet man komplexe Hydride, wie beispielsweise Natriumborhydrid, Natrium cyanoborhydrid, Lithiumborhydrid oder Lithiumaluminiumhydrid oder Aluminiumalkoholate, wie beispielsweise Aluminiumisopropylat.

Als Verdünnungsmittel zur Durchführung des erfindungsgemäßen Verfahrens (c) kommen inerte organische Lösungsmittel infrage. Hierzu gehören insbesondere aliphatische, alicyclische oder aromatische Kohlenwasserstoffe, wie beispielsweise Benzin, Benzol, Toluol, Xylol, Petrolether, Hexan oder Cyclohexan, Ether, wie Diethylether, Dioxan, Tetrahydrofuran oder Ethylenglykoldimethyl- oder -diethylether oder Alkohole, wie Methanol, Ethanol, n- oder i-Propanol, sowie n-, i-, s-oder t-Butanol.

Die Reaktionstemperaturen können bei der Durchführung des erfindungsgemäßen Verfahrens (c) in einem größeren Bereich variiert werden. Im allgemeinen arbeitet man bei Temperaturen zwischen -20 °C und +120 °C, vorzugsweise bei Temperaturen zwischen 0 °C und 100 °C.

Zur Durchführung des erfindungsgemäßen Verfahrens (c) setzt man pro Mol an substituiertem Pyridin der Formel (Ia) im allgemeinen 0.1 bis 2.0 Mol, vorzugsweise 0.3 bis 1.0 Mol an Reduktionsmittel ein.
Die Reaktionsdurchführung, Aufarbeitung und Isolierung der Reaktionsprodukte erfolgt nach allgemein üblichen Methoden.

Als Verdünnungsmittel zur Durchführung des erfindungsgemäßen Verfahrens (d) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Ketone, wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester oder Sulfoxide, wie Dimethylsulfoxid.

Das erfindungsgemäße Verfahren (d) wird vorzugsweise in Gegenwart eines geeigneten Reaktionshilfsmittels durchgeführt. Als solche kommen alle üblicherweise verwendbaren anorganischen und organischen

Basen in Frage. Vorzugsweise verwendet man Alkalimetallhydride, -hydroxide, -amide, -alkoholate, -carbonate oder -hydrogencarbonate, wie beispielsweise Natriumhydrid, Natriumamid, Natriumhydroxid, Natriummethylat, Natriumethylat, Kalium-t-butylat, Natriumcarbonat oder Natriumhydrogencarbonat oder auch tertiäre Amine, wie beispielsweise Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Das erfindungsgemäße Verfahren (d) kann gegebenenfalls auch in einem Zweiphasensystem, wie beispielsweise Wasser/Toluol oder Wasser/Dichlormethan, gegebenenfalls in Gegenwart eines Phasentransferkatalysators, durchgeführt werden. Als Beispiele für solche Katalysatoren seien genannt: Tetrabutylammoniumiodid, Tetrabutylammoniumbromid, Tributyl-methylphosphoniumbromid, Trimethyl-$C_{13}/C_{15}$-alkylammoniumchlorid, Dibenzyldimethyl-ammoniummethylsulfat, Dimethyl-$C_{12}/C_{14}$-alkylbenzylammoniumchlorid, Tetrabutylammoniumhydroxid, 15-Krone-5, 18-Krone-6, Triethylbenzylammoniumchlorid, Trimethylbenzylammoniumchlorid.

Die Reaktionstemperaturen können bei der Durchführung des erfindungsgemäßen Verfahrens (d) in einem größeren Bereich variiert werden. Im allgemeinen arbeitet man bei Temperaturen zwischen - 20 °C und + 150 °C, vorzugsweise bei Temperaturen zwischen 0 °C und + 120 °C.

Zur Durchführung des erfindungsgemäßen Verfahrens (d) setzt man pro Mol an substituiertem Pyridin der Formel (Ib) im allgemeinen 1.0 bis 5.0 Mol, vorzugsweise 1.0 bis 2.5 Mol an Alkylierungs- oder Acylierungsmittel der Formel (V) und gegebenenfalls 1.0 bis 5.0 Mol, vorzugsweise 1.0 bis 2.5 Mol an Reaktionshilfsmittel ein.

Die Reaktionsdurchführung, Aufarbeitung und Isolierung der Reaktionsprodukte erfolgt nach allgemein üblichen Methoden.

Zur Herstellung von pflanzenverträglichen Säureadditionssalzen der Verbindungen der Formel (I) kommen vorzugsweise folgende Säuren infrage: Halogenwasserstoffsäuren, wie z.B. Chlorwasserstoffsäure und Bromwasserstoffsäure, insbesondere Chlorwasserstoffsäure, ferner Phosphorsäure, Salpetersäure, Schwefelsäure, mono-, bi- und trifunktionelle Carbonsäuren und Hydroxycarbonsäuren, wie z.B. Essigsäure, Maleinsäure, Bernsteinsäure, Fumarsäure, Weinsäure, Zitronensäure, Salicylsäure, Sorbinsäure und Milchsäure, Sulfonsäuren, wie z.B. p-Toluolsulfonsäure und 1,5-Naphthalindisulfonsäure sowie Saccharin oder Thiosaccharin.

Die Säureadditionssalze der Verbindungen der Formel (I) können in einfacher Weise nach üblichen Salzbildungsmethoden, wie z.B. durch Lösen einer Verbindung der Formel (I) in einem geeigneten inerten Lösungsmittel und Hinzufügen der Säure, wie z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der Verbindungen der Formel (I) kommen vorzugsweise Salze von Metallen der II. bis IV. Haupt- und der I. und II. sowie IV. bis VIII. Nebengruppe infrage, wobei Kupfer, Zink, Mangan, Magnesium, Zinn, Eisen und Nickel beispielhaft genannt seien.

Als Anionen von Salzen kommen solche in Betracht, die sich vorzugsweise von folgenden Säuren ableiten: Halogenwasserstoffsäure, wie z.B. Chlorwasserstoffsäure und Bromwasserstoffsäure, ferner Phosphorsäure, Salpetersäure und Schwefelsäure.

Die Metallsalzkomplexe von Verbindungen der Formel (I) können in einfacher Weise nach üblichen Verfahren erhalten werden, so z.B. durch Lösen des Metallsalzes in Alkohol, z.B. Ethanol und Hinzufügen zur Verbindung der Formel (I). Man kann Metallsalz-Komplexe in bekannter Weise, z.B. durch Abfiltrieren isolieren und gegebenenfalls durch Umkristallisation reinigen.

Die erfindungsgemäßen Wirkstoffe weisen eine starke Wirkung gegen Schädlinge auf und können zur Bekämpfung von unerwünschten Schadorganismen praktisch eingesetzt werden. Die Wirkstoffe sind für den Gebrauch als Pflanzenschutzmittel, insbesondere als Fungizide einsetzbar.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezählten Oberbegriffe fallen, genannt:

Pythium-Arten, wie beispielsweise Pythium ultimum;

Phytophthora-Arten, wie beispielsweise Phytophthora infestans;

Pseudoperonospora-Arten, wie beispielsweise Pseudoperonospora humuli oder Pseudoperonospora cubensis;

Plasmopara-Arten, wie beispielsweise Plasmopara viticola;

Peronospora-Arten, wie beispielsweise Peronospora pisi oder P. brassicae;

Erysiphe-Arten, wie beispielsweise Erysiphe graminis;

Sphaerotheca-Arten, wie beispielsweise Sphaerotheca fuliginea;

Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea;
(Konidienform: Drechslera, Syn: Helminthosporium);
Cochliobolus-Arten, wie beispielsweise Cochliobolus sativus;
(Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg zur Bekämpfung von Getreidekrankheiten, wie beispielsweise gegen den Erreger des echten Getreidemehltaus (Erysiphe graminis), gegen den Erreger der Braunfleckigkeit des Weizens (Leptosphaeria nodorum), gegen den Erreger der Braunfleckenkrankheit der Gerste (Cochliobolus sativus) oder gegen der Erreger der Blattfleckenkrankheit der Gerste (Pyrenophora teres), zur Bekämpfung von Reiskrankheiten, wie beispielsweise zur Bekämpfung der Reisfleckenkrankheit (Pyricularia oryzae) oder zur Bekämpfung der Reisstengelkrankheit (Pellicularia sasakii) oder zur Bekämpfung von Krankheiten im Obst und Gemüsebau, wie beispielsweise gegen den Erreger des Bohnengrauschimmels (Botrytis cinerea) oder gegen den Erreger des Apfelschorfes (Venturia inaequalis) sowie gegen echte Mehltau- und Rostpilze in Gemüsekulturen einsetzen. Darüberhinaus besitzen die erfindungsgemäßen Wirkstoffe eine besonders breite fungizide Wirksamkeit bei in-vitro-Versuchen.

In entsprechenden Aufwandmengen besitzen die erfindungsgemäßen Wirkstoffe darüberhinaus auch eine wachstumsregulierende Wirkung bei Kulturpflanzen.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier- und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycol-ether, Alkylsulfonate, Alkylsulfate,

Arylsulfonate sowie Eiweißhydrolysate. Als Dispergiermittel kommen in Frage: z.B. Lignin-sulfitablaugen und Methylcellulose.

Es können in den Formulierungen Haftmittel wie Carboxymethylcellulose, natürliche und synthetische, pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe, wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen, wie Fungizide, Insektizide, Akarizide und Herbizide, sowie in Mischungen mit Düngemitteln und Wachstumsregulatoren.

Die Wirkstoffe können als solche, in Form ihrer Formulierungen oder den daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 %, am Wirkungsort erforderlich.

Herstellungsbeispiele

Beispiel 1

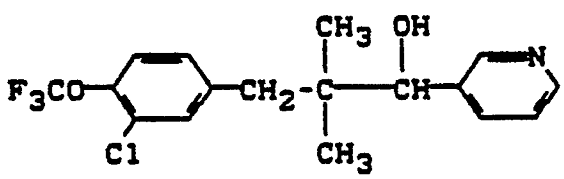

(Verfahren b)

Zu 14,8 g (0,1006 Mol) Isopropylmagnesiumbromid in 80 ml Diethylether tropft man bei Raumtemperatur unter Rühren 15,6 g (0,1 Mol) 3-Brompyridin, rührt nach beendeter Zugabe 30 Minuten bei Rückflußtemperatur, gibt dann 25,1 g (0,0671 Mol) 2,2-Dimethyl-3-(3-chlor-4-trifluormethoxy-phenyl)-propionaldehyd zu und rührt weitere 2 Stunden bei Rückflußtemperatur nach. Zur Aufarbeitung wird die abgekühlte Reaktionsmischung mit 50 ml Wasser versetzt, mit verdünnter Salzsäure auf pH 5 eingestellt, die organische Phase abgetrennt, mit Wasser gewaschen, über Natriumsulfat getrocknet und im Vakuum eingeengt. Der Rückstand wird aus Petrolether umkristallisiert. Man erhält 9,3 g (39 % der Theorie) an 2,2-Dimethyl-3-(3-chlor-4-trifluormethoxy-phenyl)-1-(3-pyridyl)-propan-1-ol vom Schmelzpunkt 116 °C.

Herstellung der Ausgangsverbindung

Beispiel IV-1

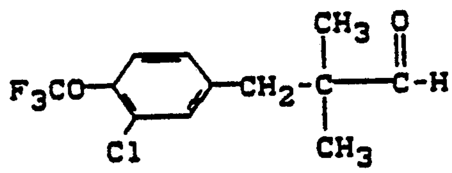

Zu einer Mischung aus 60 g (1,5 Mol) Natriumhydroxid in 140 ml Wasser und 4,5 g Tetrabutylammoniumbromid in 140 ml Toluol gibt man bei 80 °C tropfenweise unter Rühren ein Gemisch von 122,5 g (0,5 Mol) 4-Trifluormethoxy-3-chlor-benzylchlorid (vgl. z.B. EP 9787) und 51,3 g (0,712 Mol) Isobutyraldehyd, rührt anschließend 3 Stunden bei Rückflußtemperatur, kühlt ab, trennt die organische Phase ab, wäscht mit Wasser, trocknet über Natriumsulfat und destilliert im Vakuum. Man erhält 70,8 g (50,5 % der Theorie) an 2,2-Dimethyl-3-(3-chlor-4-trifluormethoxyphenyl)-propionaldehyd vom Siedepunkt 125 °C bei 6 mbar.

Beispiel 2

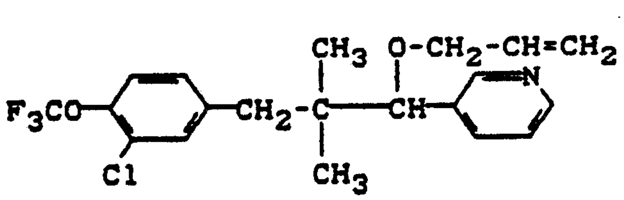

Zu 7 g (0,0195 Mol) 2,2-Dimethyl-3-(3-chlor-4-trifluormethoxyphenyl)-1-(3-pyridyl)-propan-1-ol (vgl. Bsp. 1) in 50 ml Dimethylformamid gibt man 0,76 g (0,02535 Mol) Natriumhydrid (80 %ig in Paraffin), rührt bis zum Abklingen der Gasentwicklung, gibt anschließend bei 40 °C tropfenweise unter Rühren 3,1 g (0,02535 Mol) Allylbromid zu und rührt weitere 4 Stunden bei 50 °C. Zur Aufarbeitung wird die Reaktionsmischung abgekühlt, mit Wasser versetzt und mit Dichlormethan extrahiert. Die vereinigten organischen Phasen werden über Natriumsulfat getrocknet und im Vakuum eingeengt. Der Rückstand wird durch Chromatographie an Kieselgel (Laufmittel: Essigester / Cyclohexan 3 : 1) gereinigt.

Man erhält 3,7 g (47,5 % der Theorie) an 1-Allyloxy-2,2-dimethyl-3-(3-chlor-4-trifluormethoxyphenyl)-1-(3-pyridyl)-propan als Öl.

$^1$H-NMR ($CDCl_3$/TMS) : $\delta$ = 0,79 (s, 3H); 0,90 (s, 3H) ppm.

In entsprechender Weise und gemäß den allgemeinen Angaben zur Herstellung erhält man die folgenden substituierten Pyridine der allgemeinen Formel (I):

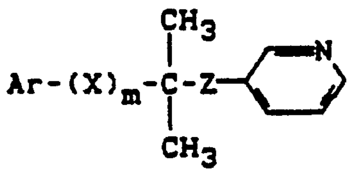 (I)

| Bsp. Nr. | Ar | $-(X)_m-$ | Z | physikalische Eigenschaften |
|---|---|---|---|---|
| 3 | 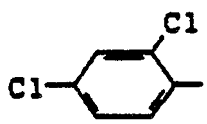 | $-CH_2-$ | 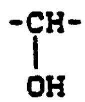 | Fp.145-146° C |
| 4 | 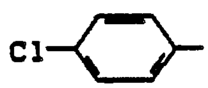 | $-CH_2-$ | 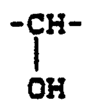 | Fp.160 °C |
| 5 | 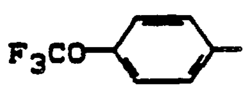 | $-CH_2-$ | 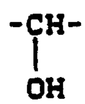 | Fp.153 °C |

| Bsp. Nr. | Ar | $-(X)_m-$ | Z | physikalische Eigenschaften |
|---|---|---|---|---|
| 6 | 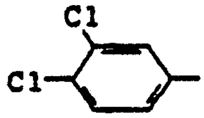 | $-CH_2-$ | 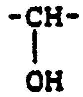 | Fp.124 °C |
| 7 | 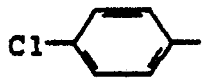 | $-CH_2-$ | 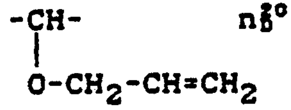 | $n_D^{20}$ 1.5271 |
| 8 | 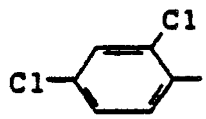 | $-CH_2-$ | 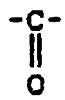 | $n_D^{20}$ 1.5840 |
| 9 | 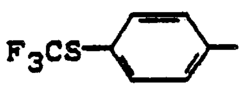 | $-CH_2-$ | 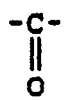 | $n_D^{20}$ 1.5396 |
| 10 | 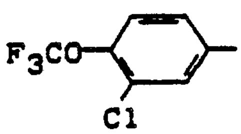 | $-CH_2-$ | 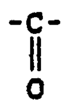 | Fp. 116° C |
| 11 | 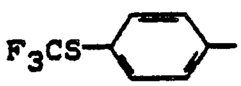 | $-CH_2-$ | 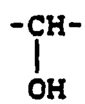 | Fp.138 °C |
| 12 | 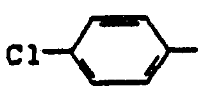 | $-CH_2-$ | 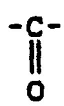 | $n_D^{20}$ 1.5711 |

| Bsp. Nr. | Ar | $-(X)_m-$ | Z | physikalische Eigenschaften |
|---|---|---|---|---|
| 13 | 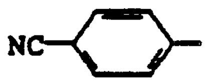 | -S- | OH<br>-CH- | Fp. 127° C |
| 14 | 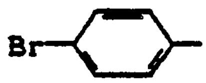 | -O- | OH<br>-CH- | Fp. 81° C |
| 15 | 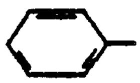 | $-CH_2$ | OH<br>-CH- | Fp. 113-114° C |
| 16 | 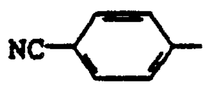 | -S- | O<br>-C- | |
| 17 | 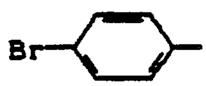 | -O- | O<br>-C- | Fp. 65° C |
| 18 | 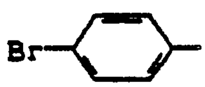 | $-CH_2-$ | OH<br>-CH- | Fp. 143° C |
| 19 | 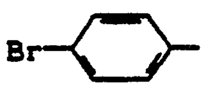 | $-CH_2-$ | O<br>-C- | $n_D^{20}$ 1.5853 |
| 20 | 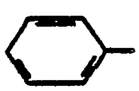 | $-CH_2-$ | O<br>-C- | $n_D^{20}$ 1.5605 |

| Bsp. Nr. | Ar | $-(X)_m-$ | Z | physikalische Eigenschaften |
|---|---|---|---|---|
| 21 | 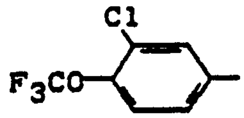 | $-CH_2-$ | O<br>‖<br>-C- | |
| 22 | 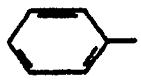 | -S- | OH<br>\|<br>-CH- | Fp. 67-69° C |
| 23 | 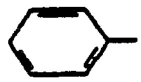 | -S- | O<br>‖<br>-C- | $n_D^{20}$ 1.5955 |
| 24 | 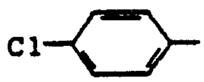 | -O- | OH<br>\|<br>-CH- | Fp. 103°C |
| 25 | 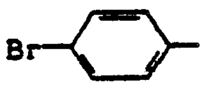 | -S- | OH<br>\|<br>-CH- | Fp. 107-109°C |
| 26 | 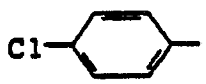 | -S- | O<br>‖<br>-C- | $n_D^{20}$ 1.6056 |
| 27 | 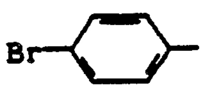 | -S- | O<br>‖<br>-C- | $n_D^{20}$ 1.6185 |
| 28 | 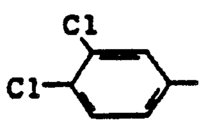 | $-CH_2-$ | O<br>‖<br>-C- | $n_D^{20}$ 1.5831 |

| Bsp. Nr. | Ar | $-(X)_m-$ | Z | physikalische Eigenschaften |
|---|---|---|---|---|
| 29 | 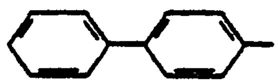 | -O- | 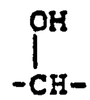 | Fp. 135-137° C |
| 30 | 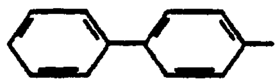 | -O- | 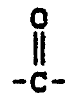 | Fp. 141-143° C |

Anwendungsbeispiele

In den folgenden Anwendungsbeispielen wurden die nachstehend aufgeführten Verbindungen als Vergleichsubstanzen eingesetzt:

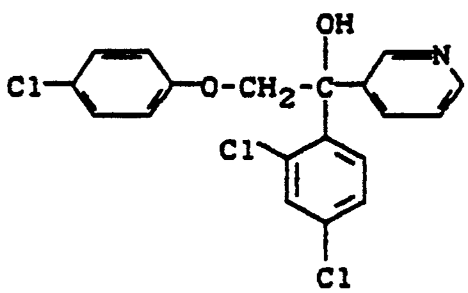

x 1/2

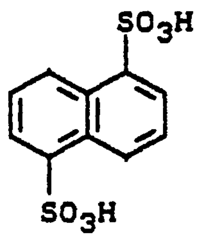

(A)

**2-(4-Chlorphenoxy)-1-(2,4-dichlorphenyl)-1-(3-pyridyl)-ethanol Naphthalindisulfonat**

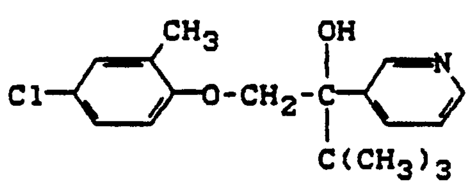

(B)

**1-(4-Chlor-2-methylphenoxy)-3,3-dimethyl-2-(3-pyridyl)-2-butanol**

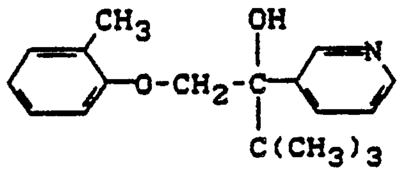

(C)

**3,3-Dimethyl-1-(2-methylphenoxy)-2-(3-pyridyl)-2-butanol**

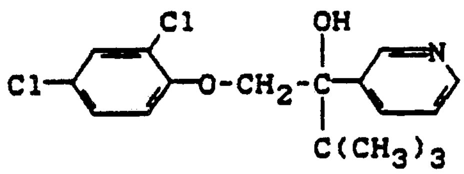

(D)

**1-(2,4-Dichlorphenoxy)-3,3-dimethyl-2-(3-pyridyl)-2-butanol**

(alle bekannt aus EP 1399).

Beispiel A

Botrytis-Test (Bohne)/protektiv

Lösungsmittel: 4,7 Gewichtsteile Aceton

Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden auf jedes Blatt 2 kleine mit Botrytis cinerea bewachsene Agarstückchen aufgelegt. Die inokulierten Pflanzen werden in einer abgedunkelten, feuchten Kammer bei 20 °C aufgestellt. 3 Tage nach der Inokulation wird die Größe der Befallsflecken auf den Blättern ausgewertet.

Eine deutliche Überlegenheit in Wirksamkeit gegenüber dem Stand der Technik zeigen in diesem Test z.B. die Verbindungen gemäß den Herstellungsbeispielen: 4 und 5.

Beispiel B

Erysiphe-Test (Gerste) / protektiv

Lösungsmittel: 100 Gewichtsteile dimethylformamid

Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp.hordei bestäubt.

Die Pflanzen werden in einem Gewächshaus bei einer Temperatur von ca. 20 °C und einer relativen Luftfeuchtigkeit von ca. 80 % aufgestellt, um die Entwicklung von Mehltaupusteln zu begünstigen.

7 Tage nach der Inokulation erfolgt die Auswertung.

Eine deutliche Überlegenheit in der Wirksamkeit gegenüber dem Stand der Technik zeigen bei diesem Test z.B. die Verbindungen gemäß den Herstellungsbeispielen: 4 und 5.

## Beispiel C

Pyricularia-Test (Reis) /protektiv

Lösungsmittel: 12,5 Gewichtsteile Aceton

Emulgator: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25 °C aufgestellt.

4 Tage nach der Inokulation erfolgt die Auswertung des Krankheitsbefalls.

Eine deutliche Überlegenheit in der Wirksamkeit gegenüber dem Stand der Technik zeigt in diesem Test z.B. die Verbindung gemäß Herstellungsbeispiel 3.

**Ansprüche**

1. Substituierte Pyridine der allgemeinen Formel (I),

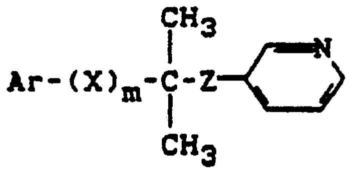

(I)

in welcher
Ar für gegebenenfalls substituiertes Aryl steht,
X für Sauerstoff, Schwefel, Sulfinyl, Sulfonyl oder für eine der Gruppen $-CH_2-$; $-O-CH_2-$; $-CH_2-O-$; $-O-CH_2-CH_2-$; $-S(O)_n-CH_2-$; $-CH_2-S(O)_n-$oder $-S(O)_n-CH_2-CH_2-$ steht,
wobei
n jeweils für eine Zahl 0, 1 oder 2 steht,
Z für eine der Gruppen $-\underset{\overset{\|}{O}}{C}-$ oder $-\underset{\overset{|}{OR}}{C}H-$ steht,
wobei
R für Wasserstoff, Alkyl, Alkenyl, Alkinyl, für gegebenenfalls substiuiertes Aralkyl oder für Alkanoyl steht und
m für eine Zahl 0 oder 1 steht,
wobei jedoch für den Fall, daß X für Sauerstoff steht, Ar nicht gleichzeitig für einfach oder zweifach durch Chlor substituiertes Phenyl oder für einfach durch Difluormethoxy substituiertes Phenyl steht, sowie deren pflanzenverträgliche Säureadditionssalze und Metallsalzkomplexe, deren Isomere und Isomerengemische.

2. Substituierte Pyridine gemäß Anspruch 1, wobei in der Formel (I)
Ar für gegebenenfalls einfach bis mehrfach, gleich oder verschieden substituiertes Phenyl steht, wobei als Substituenten infrage kommen: Halogen, Cyano, Nitro, jeweils geradkettiges oder verzweigtes Alkyl, Alkoxy, Alkylthio, Alkylsulfinyl oder Alkylsulfonyl mit jeweils 1 bis 4 Kohlenstoffatomen, geradkettiges oder verzweigtes, zweifach verknüpftes Alkandiyl mit 1 bis 10 Kohlenstoffatomen, Cycloalkyl mit 3 bis 7 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen, gegebenenfalls einfach bis mehrfach, gleich oder verschieden durch Halogen substituiertes, zweifach verknüpftes Dioxyalkylen mit 1 bis 6 Kohlenstoffatomen oder jeweils gegebenenfalls einfach bis mehrfach, gleich oder verschieden durch Halogen substituiertes Phenyl oder Phenoxy;
oder für gegebenenfalls einfach bis mehrfach, gleich oder verschieden substituiertes Naphthyl steht, wobei als Substituenten infrage kommen:
Halogen, Cyano oder geradkettiges oder verzweigtes Alkyl mit 1 bis 4 Kohlenstoffatomen;
X für Sauerstoff, Schwefel, Sulfinyl, Sulfonyl oder für eine der Gruppen $-CH_2-$; $-O-CH_2-$; $-CH_2-O-$; $-O-CH_2-CH_2-$; $-S(O)_n-CH_2-$; $-CH_2-S(O)_n-$oder $-S(O)_n-CH_2-CH_2-$ steht,
wobei
n jeweils für eine Zahl 0, 1 oder 2 steht,
Z für eine der Gruppen $-\underset{\overset{\|}{O}}{C}-$ oder $-\underset{\overset{|}{OR}}{C}H-$ steht,
wobei
R für Wasserstoff, für geradkettiges oder verzweigtes Alkyl mit 1 bis 6 Kohlenstoffatomen, für jeweils geradkettiges oder verzweigtes Alkenyl oder Alkinyl mit jeweils 3 bis 6 Kohlenstoffatomen oder für einfach bis mehrfach, gleich oder verschieden substituiertes Benzyl steht, wobei als Substituenten infrage kommen: Halogen, insbesondere Fluor, Chlor oder Brom oder jeweils geradkettiges oder verzweigtes Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen oder für geradkettiges oder verzweigtes Alkanoyl mit 1 bis 7 Kohlenstoffatomen steht und
m für eine Zahl 0 oder 1 steht,

wobei jedoch für den Fall, daß X für Sauerstoff steht, Ar nicht gleichzeitig für einfach oder zweifach durch Chlor substituiertes Phenyl oder für einfach durch Difluormethoxy substituiertes Phenyl steht, sowie deren Säureadditionssalze, Metallsalzkomplexe, Isomeren und Isomerengemische.

3. Substituierte Pyridine gemäß Anspruch 1, wobei in der Formel (I)

Ar für gegebenenfalls ein- bis dreifach, gleich oder verschieden substituiertes Phenyl steht, wobei als Substituenten infrage kommen: Fluor, Chlor, Brom, Cyano, Nitro, Methyl, Ethyl, n-oder i-Propyl, n-, i-, s- oder t-Butyl, Methoxy, Ethoxy, n- oder i-Propoxy, Methylthio, Ethylthio, Propan-1,3-diyl, Butan-1,4-diyl, Pentan-1,5-diyl, Dimethyl-propan-1,3-diyl, Tetramethylpropan-1,3-diyl, Dimethyl-butan-1,4-diyl, Tetramethylbutan-1,4-diyl, Cyclopropyl, Cyclobutyl, Cyclopentyl, Cylohexyl, Trifluormethyl, Dichlorfluormethyl, Difluorchlormethyl, Difluorbrommethyl, Trichlormethyl, Fluormethyl, Difluormethyl, Trifluorethyl, Tetrafluorethyl, Trifluorchlorethyl, Pentafluorethyl, Trifluormethoxy, Difluormethoxy, Fluormethoxy, Dichlorfluormethoxy, Difluorchlormethoxy, Difluorbrommethoxy, Trichlormethoxy, Trifluorethoxy, Tetrafluorethoxy, Pentafluorethoxy, Trifluorchlorethoxy, Trifluordichlorethoxy, Difluortrichlorethoxy, Pentachlorethoxy, Trifluormethylthio, Difluormethylthio, Fluormethylthio, Difluorchlormethylthio, Dichlorfluormethylthio, Difluorethylthio, Difluorbrommethylthio, Trichlormethylthio, Trifluorethylthio, Tetrafluorethylthio, Pentafluorethylthio, Trifluorchlorethylthio, Trifluordichlorethylthio, Penta chlorethylthio, Methylsulfinyl, Trifluormethylsulfinyl, Dichlorfluormethylsulfinyl, Difluorchlormethylsulfinyl, Fluormethylsulfinyl, Difluormethylsulfinyl, Methylsulfonyl, Trifluormethylsulfonyl, Dichlorfluormethylsulfonyl, Difluorchlormethylsulfonyl, Fluormethylsulfonyl, Difluormethylsulfonyl, Methoxycarbonyl, Ethoxycarbonyl, Methoximinomethyl, Ethoximinomethyl, Methoximinoethyl, Ethoximinoethyl, Difluordioxymethylen, Tetrafluordioxyethylen, Trifluordioxyethylen, Difluordioxyethylen, Dioxymethylen, Dioxyethylen, jeweils gegebenenfalls ein- bis dreifach durch Fluor oder Chlor substituiertes Phenyl oder Phenoxy;

oder für jeweils gegebenenfalls ein- bis dreifach, gleich oder verschieden substituiertes α-Naphthyl oder β-Naphthyl steht, wobei als Substituenten jeweils infrage kommen: Fluor, Chlor, Brom, Cyano, Methyl, Ethyl, n- oder i-Propyl;

X für Sauerstoff, Schwefel, Sulfinyl, Sulfonyl oder für eine der Gruppen $-CH_2-$; $-O-CH_2-$; $-CH_2-O-$; $-O-CH_2-CH_2-$; $-S(O)_n-CH_2-$; $-CH_2-S(O)_n-$oder $-S(O)_n-CH_2-CH_2-$ steht,

wobei

n jeweils für eine Zahl 0, 1 oder 2 steht,

Z für eine der Gruppen $-\underset{\overset{\|}{O}}{C}-$ oder $-\underset{\overset{|}{OR}}{CH}-$ steht,

wobei

R für Wasserstoff, Methyl, Ethyl, n- oder i-Propyl, n-, i-, s- oder t-Butyl, Allyl, n- oder i-Butenyl, Propargyl, n- oder i-Butinyl, für gegebenenfalls ein- bis dreifach, gleich oder verschieden durch Fluor, Chlor, Brom, Methyl, Ethyl, n-oder i-Propyl, n-, i-, s- oder t-Butyl, Methoxy, Ethoxy, n- oder i-Propoxy, Methylthio, Ethylthio, Trifluormethyl, Trifluormethoxy oder Trifluormethylthio substituiertes Benzyl oder für Acetyl oder Propionyl steht und

m für eine Zahl 0 oder 1 steht,

wobei jedoch für den Fall, daß X für Sauerstoff steht, Ar nicht gleichzeitig für einfach oder zweifach durch Chlor substituiertes Phenyl oder für einfach durch Difluormethoxy substituiertes Phenyl steht, sowie deren Säureadditionssalze, Metallsalzkomplexe, deren Isomere und Isomerengemische.

4. Substituierte Pyridine gemäß Anspruch 1, wobei in der Formel (I)

Ar für gegebenenfalls ein- bis dreifach, gleich oder verschieden substituiertes Phenyl steht, wobei als Substituenten infrage kommen: Fluor, Chlor, Brom, Cyano, Nitro, Methyl, Ethyl, t-Butyl, Methoxy, Ethoxy, n- oder i-Propoxy, Methylthio, Cyclopropyl, Cyclopentyl, Cyclohexyl, Propan-1,3-diyl, Butan-1,4-diyl, Pentan-1,5-diyl, 1,1,3,3-Tetramethyl-propan-1,3-diyl, Trifluormethyl, Trifluormethoxy, Trifluormethylthio, Difluormethyl, Difluormethoxy, Difluormethylthio, Difluorchlormethyl, Difluorchlormethoxy, Difluorchlormethylthio, Dichlorfluormethyl, Dichlorfluormethoxy, Dichlorfluormethylthio, Methoximinomethyl, Ethoximinomethyl, Methoximinoethyl, Ethoxyiminoethyl, Difluordioxymethylen, Trifluordioxyethylen, Tetrafluordioxyethylen, Phenoxy oder Phenyl; außerdem für α-Naphthyl oder β-Naphthyl steht,

X für Schwefel, für eine $-CH_2$-Gruppe; eine $-O-CH_2$-Gruppe; eine $-S-CH_2$-Gruppe, eine $-O-CH_2-CH_2$-Gruppe oder eine $-S-CH_2-CH_2$-Gruppe steht,

Z für eine $-\underset{\overset{\|}{O}}{C}-$ Gruppe oder eine $-\underset{\overset{|}{OH}}{CH}-$ Gruppe steht,

und

m für eine Zahl 0 oder 1 steht, sowie deren Säureadditionssalze, Metallsalzkomplexe, deren Isomere und Isomerengemische.

5. Substituierte Pyridine gemäß Anspruch 1, wobei in der Formel (I)
Ar für ein- bis dreifach, gleich oder verschieden substituiertes Phenyl steht, wobei mindestens ein Substituent für einen der folgenden Reste steht: Methylthio, Difluormethylthio, Trifluormethylthio, Difluorchlormethylthio, Dichlorfluormethylthio, Propan-1,3-diyl, Butan-1,4-diyl, Pentan-1,5-diyl, 1,1,3,3-Tetramethylpropan-1,3-diyl, Cyclopropyl, Cyclopentyl, Cyclohexyl, Dioxymethylen, Dioxyethylen, Difluordioxymethylen, Trifluordioxyethylen, Tetrafluordioxyethylen, Methoximinomethyl, Methoximinoethyl, Ethoximinomethyl, Ethoximinoethyl, gegebenenfalls ein- bis dreifach durch Fluor und/oder Chlor substituiertes Phenoxy oder ein- bis dreifach durch Fluor und/oder Chlor substituiertes Phenyl; und wobei als weitere Substituenten infrage kommen: Fluor, Chlor, Brom, Cyano, Nitro, Methyl, Ethyl, t-Butyl, Methoxy, Ethoxy, n- oder i-Propoxy, Trifluormethyl, Trifluor methoxy, Difluormethyl, Difluormethoxy, Difluorchlormethyl, Difluorchlormethoxy, Dichlorfluormethyl, Dichlorfluormethoxy oder Phenyl;
außerdem für α-Naphthyl oder β-Naphthyl steht,
X für Sauerstoff steht,
Z für eine $-\underset{\underset{O}{\|}}{C}-$-Gruppe oder eine $-\underset{\underset{OH}{|}}{CH}-$ - Gruppe steht,
und
m für eine Zahl 0 oder 1 steht, sowie deren Säureadditionssalze, Metallsalzkomplexe, deren Isomere und Isomerengemische.

6. Verfahren zur Herstellung von substituierten Pyridinen der allgemeinen Formel (I),

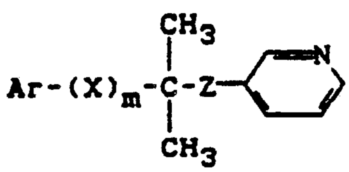

(I)

in welcher
Ar für gegebenenfalls substituiertes Aryl steht,
X für Sauerstoff, Schwefel, Sulfinyl, Sulfonyl oder für eine der Gruppen $-CH_2-$; $-O-CH_2-$; $-CH_2-O-$; $-O-CH_2-CH_2-$; $-S(O)_n-CH_2-$; $-CH_2-S(O)_n-$oder $-S(O)_n-CH_2-CH_2-$ steht,
wobei
n jeweils für eine Zahl 0, 1 oder 2 steht,
Z für eine der Gruppen $-\underset{\underset{O}{\|}}{C}-$ oder $-\underset{\underset{OR}{|}}{CH}-$ steht,
wobei
R für Wasserstoff, Alkyl, Alkenyl, Alkinyl, für gegebenenfalls substiuiertes Aralkyl oder für Alkanoyl steht und
m für eine Zahl 0 oder 1 steht,
wobei jedoch für den Fall, daß X für Sauerstoff steht, Ar nicht gleichzeitig für einfach oder zweifach durch Chlor substituiertes Phenyl oder für einfach durch Difluormethoxy substituiertes Phenyl steht, sowie deren Säureadditionssalze, Metallsalzkomplexe, deren Isomere und Isomerengemische, dadurch gekennzeichnet, daß man

(a) substituierte Pyridine der Formel (Ia),

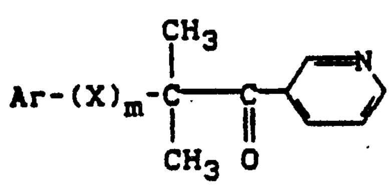

(Ia)

in welcher
Ar, X und m die oben angegebene Bedeutung haben, erhält,
wenn man Ester der Formel (II),

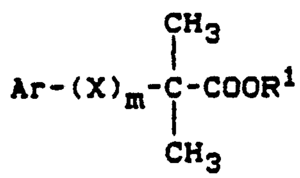
(II)

in welcher
$R^1$ für Alkyl steht und
Ar, X und m die oben angegebene Bedeutung haben,
mit Pyridyl-Grignard-Verbindungen der Formel (III),

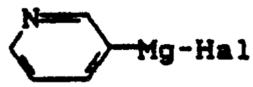
(III)

in welcher
Hal für Halogen steht,
gegebenenfalls in Gegenwart eines Verdünnungsmittels umsetzt;
(b) substituierte Pyridine der Formel (Ib),

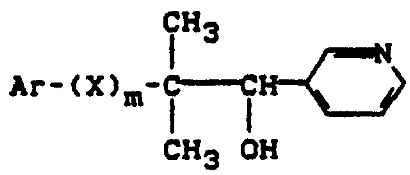
(Ib)

in welcher
Ar, X und m die oben angegebene Bedeutung haben, erhält,
wenn man Aldehyde der Formel (IV),

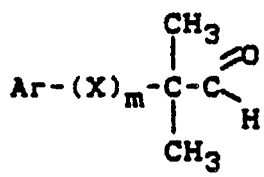
(IV)

in welcher
Ar, X und m die oben angegebene Bedeutung haben,
mit Pyridyl-Grignard-Verbindungen der Formel (III),

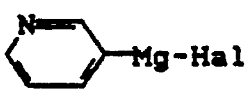
(III)

in welcher
Hal für Halogen steht,
gegebenenfalls in Gegenwart eines Verdünnungsmittels umsetzt;
(c) substituierte Pyridine der Formel (Ib),

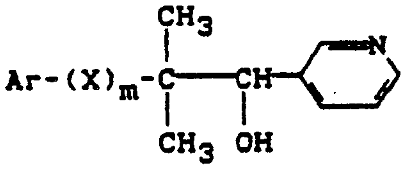

(Ib)

in welcher
Ar, X und m die oben angegebene Bedeutung haben,
alternativ auch erhält, wenn man substituierte Pyridine der Formel (Ia),

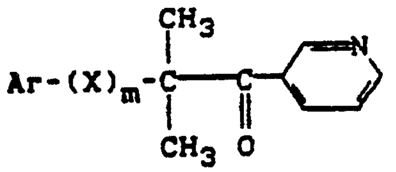

(Ia)

in welcher
Ar, X und m die oben angegebene Bedeutung haben,
mit einem Reduktionsmittel, gegebenenfalls in Gegenwart eines Verdünnungsmittels umsetzt;

(d) substituierte Pyridine der Formel (Ic),

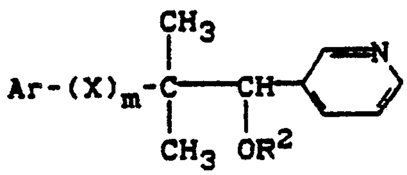

(Ic)

in welcher
$R^2$ für Alkyl, Alkenyl, Alkinyl, für gegebenenfalls substituiertes Aralkyl oder für Alkanoyl steht und
Ar, X und m die oben angegebene Bedeutung haben, erhält,
wenn man substituierte Pyridine der Formel (Ib),

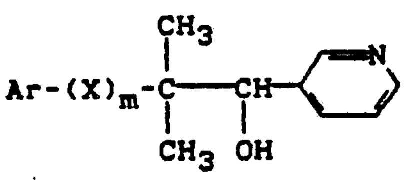

(Ib)

in welcher
Ar, X und m die oben angegebene Bedeutung haben,
mit Alkylierungs- oder Acylierungsmitteln der Formel (V),

$R^2$-A (V)

in welcher
A für eine elektronenanziehende Abgangsgruppe steht und
$R^2$ die oben angegebene Bedeutung hat, gegebenenfalls in Gegenwart eines Verdünnungsmittels und gegebenenfalls in Gegenwart eines Reaktionshilfsmittels umsetzt und gegebenenfalls anschließend eine Säure oder ein Metallsalz addiert.

7. Schädlingsbekämpfungsmittel, gekennzeichnet durch einen Gehalt an mindestens einem substituierten Pyridin der Formel (I) nach den Ansprüchen 1 und 6.

8. Verwendung von substituierten Pyridinen der Formel (I) nach den Ansprüchen 1 und 6 zur Bekämpfung von Schädlingen.

9. Verfahren zur Bekämpfung von Schädlingen, dadurch gekennzeichnet, daß man substituierte Pyridine der Formel (I) nach den Ansprüchen 1 und 6 auf Schädlinge und/oder ihren Lebensraum einwirken läßt.

10. Verfahren zur Herstellung von Schädlingsbekämpfungsmitteln, dadurch gekennzeichnet, daß man substituierte Pyridine der Formel (I) nach den Ansprüchen 1 und 6 mit Streckmitteln und/oder oberflächenaktiven Mitteln vermischt.